# EUROPEAN PATENT SPECIFICATION

(11) **EP 2 545 384 B1**
(45) Date of publication and mention of the grant of the patent: **13.12.2017**
(21) Application number: 11707354.4
(22) Date of filing: 09.03.2011
(51) Int. Cl.: C07K 2/00, G01N 33/68

(54) **METHOD FOR DETERMINING THE CONCENTRATION OF A PEPTIDE**
VERFAHREN ZUR BESTIMMUNG DER KONZENTRATION EINES PEPTIDS
MÉTHODE PERMETTANT DE DÉTERMINER LA CONCENTRATION D'UN PEPTIDE

(30) Priority: 12.03.2010 EP 10002664
(43) Date of publication of application: 16.01.2013
(73) Proprietor: JPT Peptide Technologies GmbH, 12489 Berlin (DE)
(72) Inventor: ZERWECK, Johannes, 12526 Berlin (DE); SCHUTKOWSKI, Mike, 06268 Querfurt (DE); WENSCHUH, Holger, 12555 Berlin (DE)
(74) Representative: Schnappauf, Georg
(86) International application number: PCT/EP2011/001167
(87) International publication number: WO 2011/110341

(56) References cited:
- EP-A1- 2 124 060
- US-A1- 2004 033 530
- JPT Peptide Technologies GmbH: "SpikeTidesTM: Low Cost proteotypic Peptides - Light - Heavy - Quantitfied", Press release , 16 December 2009 (2009-12-16), XP002638903, Retrieved from the Internet: URL:http://dev.jpt.glutrot.de/news/press_r eleases/single_view/?tx_ttnews[tt_news]=22 &cHash=8c5e30d0e32e8d586928dbf055f7208e [retrieved on 2011-05-26]
- JPT Peptide Technologies GmbH: "SpikeTidesTM - Synthetic Proteotypic Peptides : light - heavy -quantified", , XP002638904, Retrieved from the Internet: URL:http://www.jpt.com/?id=42 [retrieved on 2011-05-26]
- "Final Program - iSBTc 24th Annual Meeting", , 29 October 2009 (2009-10-29), XP55094966, Retrieved from the Internet: URL:http://www.sitcancer.org/UserFiles/fil e/iSBTc-AM09-Final-Program.pdf [retrieved on 2014-01-07]

## Description

The present invention is related to an internal peptide standard, a composition comprising a plurality of different species of the internal peptide standard, the use of the internal peptide standard in a method for determining the presence and/or quantity of a target polypeptide in at least one mixture of different polypeptides and/or quantity of a purified polypeptide, and methods for determining the presence and/or quantity of a target polypeptide of which a first peptide is a proteotypic peptide, in at least one mixture of different polypeptides.

There is a need in the art to provide novel methods for the quantification of proteins and modified proteins from cell lysates. Furthermore, there is a need to provide novel methods for the accurate comparison of protein expression levels between cells in two different states, particularly for comparison of low abundance proteins.

The current standard for protein detection and more specifically protein quantification is based on immunoreactive detection (Western analysis). However, this technique requires the availability of an appropriately specific antibody. In addition, many antibodies only recognize proteins in an unfolded (denatured) form, cross-reactivity can be severely limiting, and quantification is generally relative.

The development of methods and instrumentation for automated, data-dependent electrospray ionization (ESI) tandem mass spectrometry (MS/MS) in conjunction with microcapillary liquid chromatography (LC) and database searching has significantly increased the sensitivity and speed of the identification of gel-separated proteins. Microcapillary LC-MS/MS has been used successfully for the large-scale identification of individual proteins directly from mixtures without gel electrophoretic separation (Link et al., 1999; Opitek et al., 1997). However, while these approaches dramatically accelerate protein identification, quantities of the analyzed proteins cannot be easily determined, and these methods have not been shown to substantially alleviate the dynamic range problem also encountered by the 2DE/MS/MS approach. Therefore, low abundance proteins in complex samples are also difficult to analyze by the microcapillary LC/MS/MS method without their prior enrichment.

Another methodology has recently been described. ICAT reagent technology makes use of a class of chemical reagents called isotope coded affinity tags (ICAT). These reagents exist in isotopically heavy and light forms which are chemically identical with the exception of eight deuterium or hydrogen atoms, respectively. Proteins from two cells lysates can be labeled independently with one or the other ICAT reagent at cysteinyl residues. After mixing and proteolysing the lysates, the ICAT-labeled peptides are isolated by affinity to a biotin molecule incorporated into each ICAT reagent. ICAT-labeled peptides are analyzed by LC- MS/MS where they elute as heavy and light pairs of peptides. Quantification is performed by determining the relative expression ratio relating to the amount of each ICAT-labeled peptide pair in the sample.

Identification of each ICAT-labeled peptide is performed by a second stage of mass spectrometry (MS/MS) and sequence database searching. The end result is relative protein expression ratios on a large scale. The major drawbacks to this technique are 1) quantification is only relative; 2) specialized chemistry is required, and 3) database searches are hindered by the presence of the large ICAT reagent molecule, 4) relative amounts of post-translationally modified (e. g., phosphorylated) proteins are transparent to analysis.

International patent application WO 03/016861 discloses a method for absolute quantification of proteins directly from cell lysates whereby an internal peptide standard is used. The method requires the addition of a known amount of the internal standard peptide.

EP 2 124 060 A1 describes a method for high throughput peptide/protein assay generation and assays generated therewith.

US 2004/0033530 A1 discloses methods for high throughput determination of the identity, quantity and solubility profile of a plurality of recombinant proteins.

The problem underlying the instant invention is to provide a method for determining the presence of a target polypeptide in a mixture of different polypeptides. A further problem underlying the instant invention is to provide a method for determining the quantity of a target polypeptide in a mixture of different polypeptides.

A further problem underlying the instant invention is to provide a method for the generation of an internal peptide standard, whereby the internal peptide standard is particularly suitable for use in a method for determining the presence and/or quantity of a target polypeptide in a mixture of different polypeptides.

A still further problem underlying the instant invention is to provide an internal peptide standard and a panel of internal standard peptides, respectively, which is particularly suitable for use in a method for determining the presence and/or quantity of a target polypeptide in a mixture of different polypeptides.

These and other problems underlying the present invention are solved by the subject matter of the attached independent claims. Preferred embodiments may be taken from the attached dependent claims and the following description.

In a first aspect, the present invention provides an internal peptide standard comprising a first tag to which is coupled a first peptide comprising a mass label and an amino acid sequence, wherein the C-terminal end of the amino acid sequence of the first peptide corresponds to the C-terminal end generated by a sequence-specific hydrolysis of an amide bond, ester bond or thioester bond of a peptide and wherein the first tag comprises a label and is removable by a means selected from the group comprising chemical cleavage, enzymatic cleavage and physical cleavage, the label comprising meta-nitro-tyrosine. In one embodiment, the removal of the first tag is sequence-specific. In one embodiment, the mass label is selected from the group comprising C isotopes and N isotopes. In one embodiment, the internal peptide standard comprises a second tag. The second tag may comprise an anchor moiety, such as biotin, desthiobiotin, avidin, streptavidin or a chemical group allowing chemoselective reaction with the appropriate reactive function on the surface. In one embodiment, a linker connects the anchor moiety and the first peptide. In one embodiment, the first tag is coupled to the C-terminal end of the first peptide. In one embodiment, the second tag is coupled to the N-terminal end of the first peptide.

In a second aspect, the present invention provides a composition comprising a plurality of different species of the internal peptide standard according to the first aspect, wherein said different species of said internal peptide standard differ from each other in the first tag and/or in the amino acid sequence of the first peptide.

In a third aspect, the present invention provides the use of the internal peptide standard according to the first aspect in a method for determining the presence and/or quantity of a target polypeptide in at least one mixture of different polypeptides and/or quantity of a purified polypeptide.

In a fourth aspect, the present invention provides a method for determining the presence and/or quantity of a target polypeptide of which a first peptide is a proteotypic peptide, in at least one mixture of different polypeptides, comprising:
a) providing a mixture of different polypeptides preferably containing the target polypeptide;
b) adding a quantity of (i) an internal peptide standard according to the first aspect or (ii) a plurality of different species of said internal peptide standard, said different species of said internal peptide standard differing from each other in the first tag and/or in the amino acid sequence of the first peptide, thereby generating a spiked mixture;
c) treating the spiked mixture with a means for sequence-specific hydrolysis to generate a plurality of peptides from the mixture of different polypeptides and to cleave off the first tag from the internal peptide standard/standards thus releasing the first peptide, whereby the plurality of peptides comprises the proteotypic peptide derived from the different polypeptides and the first peptide released from the internal standard;
d) determining the amount of the first tag contained in the spiked mixture and therefrom the quantity of the internal standard/standards added in step b);
e) determining the ratio of the first peptide to the proteotypic peptide derived from the different polypeptides, each generated in step c); and
f) calculating from the ratio and the quantity of the internal standard/standards determined in step d) the quantity of the target polypeptide in the mixture of different polypeptides.

In a fifth aspect, the present invention provides a method for determining the presence and/or quantity of a target polypeptide of which a first peptide is a proteotypic peptide, in at least one mixture of different polypeptides, comprising:
a) providing a mixture of different polypeptides preferably containing the target polypeptide;
b) providing (i) an internal peptide standard according to the first aspect or (ii) a plurality of different species of said internal peptide standard, said different species of said internal peptide standard differing from each other in the first tag and/or in the amino acid sequence of the first peptide, treating the internal peptide standard or the plurality of different species of said internal peptide standard with a means for sequence-specific hydrolysis to cleave off the first tag from the internal peptide standard or the plurality of different species of said internal peptide standard thus releasing the first peptide;
either
ca1) adding the reaction mixture obtained in step b) to the mixture of different polypeptides of step a) and
ca2) treating the mixture obtained in step ca1) with a means for sequence-specific hydrolysis to generate a plurality of peptides from the mixture of different polypeptides, whereby the plurality of peptides comprises the proteotypic peptide;
or
cb1) treating the mixture of different polypeptides of step a) with a means for sequence-specific hydrolysis to generate a plurality of peptides from the mixture of different polypeptides, whereby the plurality of peptides comprises the proteotypic peptide; and
cb2) adding the reaction mixture obtained in step b) to the mixture obtained in step cb1)
whereby after step ca2) and cb2), respectively, a spiked mixture is obtained;
d) determining the amount of the first tag contained in the spiked mixture and therefrom the quantity of the internal standard/standards added in step ca1) or cb2);
e) determining in the spiked mixture the ratio of the first peptide to the proteotypic peptide derived from the different polypeptides; and
f) calculating from the ratio and the quantity of the internal standard/standards determined in step d) the quantity of the target polypeptide in the mixture of different polypeptides.

In a sixth aspect, the present invention provides a method for determining the presence and/or quantity of a target polypeptide of which a first peptide is a proteotypic peptide, in at least one mixture of different polypeptides, comprising:
a) providing (i) an internal peptide standard according to the first aspect or (ii) a plurality of different species of said internal peptide standard, said different species of said internal peptide standard differing from each other in the first tag and/or in the amino acid sequence of the first peptide, treating the internal peptide standard or the plurality of different species of said internal peptide standard with a means for sequence-specific hydrolysis to cleave off the first tag from the internal peptide standard or the plurality of different species of said internal peptide standard thus releasing the first peptide;
b) optionally removing the means for sequence-specific hydrolysis;
c) determining the amount of the first tag contained in the mixture obtained from step a) or b) and therefrom the amount of the internal standard contained in said mixture;
d) providing a mixture of different polypeptides preferably containing the target polypeptide;
either
ea1) adding a part of the reaction mixture obtained in step a) or b) to the mixture of different polypeptides of step d) and
ea2) treating the mixture obtained in step ea1) with a means for sequence-specific hydrolysis to generate a plurality of peptides from the mixture of different polypeptides, whereby the plurality of peptides comprises the proteotypic peptide;
or
eb1) treating the mixture of different polypeptides of step d) with a means for sequence-specific hydrolysis to generate a plurality of peptides from the mixture of different polypeptides, whereby the plurality of peptides comprises the proteotypic peptide; and
eb2) adding a part of the mixture obtained in step a) or b) to the mixture obtained in step eb1);
whereby after step ea2) and eb2), respectively, a spiked mixture is obtained;
f) determining in the spiked mixture the ratio of the first peptide to the proteotypic peptide derived from the different polypeptides; and
g) calculating from the ratio and the quantity of the internal standard/standards the quantity of the target polypeptide in the mixture of different polypeptides.

In one embodiment of the fourth to sixth aspects, the ratio of the first peptide released from the internal peptide standard to the proteotypic peptide derived from the different polypeptides is determined by mass spectrometry, preferably multistage mass spectrometry. In one embodiment of the fourth to sixth aspects, the means for sequence-specific hydrolysis is selected from a proteolytic enzyme, a sequence-specific chemical reaction or a sequence-specific physical treatment.

The present inventors have surprisingly found that it is possible to determine the presence of a polypeptide in a mixture of different polypeptides using a particular form of an internal standard. More specifically, the present inventors have surprisingly found that a labelled internal standard is suitable for such purpose and that such labelled internal standard does not need to be quantified prior to adding it to a mixture of different polypeptides, but that it is sufficient to determine its quantity upon having it added to the mixture of different polypeptides. It is, however, also within the present invention that the labelled internal standard is quantified prior to adding it to a mixture of different polypeptides.

The methods for determining the presence and/or quantity of a target polypeptide in at least one mixture of different polypeptides of the present invention are based on the concept of proteotypic peptides.

A proteotypic peptide as used herein is a peptide fragment of a polypeptide of interest which is unique for the polypeptide in a mixture of polypeptides. Because of this, the identification and quantification, respectively, of the proteotypic peptide is directly and unambiguously correlated with the presence and quantity of the polypeptide of interest in the individual mixture of polypeptides. The amino acid sequence of a proteotypic peptide is preferably contained only once in the polypeptide of interest, although also amino acid sequences which are contained in a polypeptide of interest several times, may be suitable to be the amino acid of a proteotypic peptide. In such case, the only prerequisite for the suitability of this kind of amino acid sequence to act and thus be useful as an amino acid sequence of a proteotypic peptide is that there is a defined relationship or stoichiometry between the number of copies of such amino acid sequence contained in the amino acid sequence of the polypeptide of interest.

However, it is also within the present invention that the amino acid sequence of a proteotypic peptide is contained in two or more polypeptides in a mixture of polypeptides. In such case, the proteotypic peptide is correlated with the two or more polypeptides in the mixture of polypeptides. The quantification of the proteotypic peptide in such case is a quantification of the overall amount of the two or more polypeptides contained in the mixture sharing the amino acid sequence of the proteotypic peptide.

Preferably, the length of a proteotypic peptide is from 4 to 40 amino acid residues, preferably 6 to 25 amino acid residues and more preferably 8 to 15 amino acid residues and most preferable 8-12 amino acid residues.

The practicing of the various methods for determining the presence and/or quantity of a target polypeptide of which a first peptide is a proteotypic peptide, in at least one mixture of different polypeptides, preferably requires the use of an internal peptide standard. Thus, described herein is a method for preparing an internal peptide standard. In a preferred embodiment the thus generated standard or panel of standards is used in or is for use in a method for determining the presence and/or quantity of a target polypeptide in at least one mixture of different polypeptides and/or quantity of a purified polypeptide, whereby preferably such method is a method for determining the presence and/or quantity of a target polypeptide in at least one mixture of different polypeptides and/or quantity of a purified polypeptide of the invention.

The method for preparing an internal peptide standard may comprise the following steps
a) providing a first tag, and
b) coupling of a first peptide to the first tag, whereby the first peptide comprises an amino acid sequence,
whereby the C-terminal end of the amino acid sequence of the first peptide corresponds to the C-terminal end generated by a sequence-specific hydrolysis of an amide bond, ester bond or thioester bond. Preferably, the bond is an amide bond and more specifically of an amide bond of a peptide. In this embodiment, the first peptide is provided as the product of a synthesis, typically in the form of a full length peptide. Methods for the synthesis of peptides are known to the person skilled in the art and, among others, described in Pennington, 1994, Peptide Synthesis Protocols (Methods in Molecular Biology); Benoiton, 2005, Chemistry of Peptide Synthesis.

Alternatively, the method for preparing an internal peptide standard according to invention, may comprise the following steps
a) providing a first tag and
b) coupling to the first tag the amino acids forming the first peptide comprising an amino acid sequence,
whereby the C-terminal end of the amino acid sequence of the first peptide corresponds to the C-terminal end generated by a sequence-specific hydrolysis of an amide bond, ester bond or thioester bond preferably of an amide bond of a peptide. In this embodiment, the individual amino acids forming the first peptide are subsequently attached to the first tag. It is also within the present invention that groups of amino acids forming part of the amino acid sequence of the first peptide are attached to the first tag or to the first tag once one or several of the amino acids forming part of the amino acid sequence of the first peptide have been attached to the first tag.

As used herein, the term C-terminal end of an amino acid sequence preferably means the last, i.e. most C-terminal amino acid residue of an amino acid sequence.

The sequence-specific hydrolysis is performed by means of a reaction selected from the group comprising a reaction of a proteolytic enzyme, a sequence-specific chemical reaction or a sequence-specific physical treatment. This kind of reactions is known to a person skilled in the art, see, e.g., Handbook of Proteolytic Enzymes, Academic Press, 1998; Kaiser and Metzka, 1999.

In the embodiment where the sequence-specific hydrolysis is performed by means of a proteolytic enzyme, the proteolytic enzyme is selected from the group comprising hydrolases especially endoproteinases like trypsin yielding a C-terminal arginine and lysine residue, thrombin mainly yielding a C-terminal arginine residue, V8 protease yielding a C-terminal glutamic acid residue, prolyl endopeptidase yielding a C-terminal proline residue, subtilisin and chymotrypsin mainly yielding a C-terminal phenylalanine, tyrosine, tryptophan, methionine or leucine residue and elastase yielding a C-terminal alanine, glycine serine or valine residue.

In the embodiment where the sequence-specific hydrolysis is performed by means of a sequence-specific chemical reaction, the sequence specific chemical reaction is preferably selected from the group comprising treatment with cyanogens halides like cyanogen bromide yielding a C-terminal homoserine lactone and selective acidic hydrolysis of aspartyl-prolyl bonds yielding a C-terminal aspartic acid residue.

In the embodiment where the sequence-specific hydrolysis is performed by means of a sequence-specific physical treatment, such sequence-specific physical treatment is preferably selected from ionization-caused fragmentation reactions under high vacuum which, for example, may occur in a mass spectrometer (Pfeifer, T., Schierhorn, A., Friedemann, R., Jakob, M., Frank, R., Schutkowski, M., & Fischer G. (1997) Specific Fragmentation of Thioxo Peptides Facilitates the Assignment of the Thioxylated Amino Acid. J. Mass Spec. 32, 1064-1071).

In a preferred embodiment, and preferably irrespective of which kind of means is used for the sequence-specific hydrolysis, the C-terminal end is an amino acid residue. Preferably such amino acid residue is selected from the group comprising arginine, lysine, glutamic acid and proline.

The first peptide comprises an amino acid sequence. In an embodiment, the first peptide consists of an amino acid sequence. To said first peptide a first tag is attached. Preferably, such first tag is attached to the first peptide by a covalent bond. In an embodiment, there is a second tag attached to the first peptide. In an embodiment, both a first tag and a second tag are attached to the first peptide. In the embodiment where a first tag is attached to the first peptide and a second tag is attached to the first peptide, preferably the first tag is attached to the C-terminal amino acid residue of the first peptide, and the second tag is attached to the N-terminal amino acid residue of the first peptide. For example, the second tag may be coupled to the N-terminal amino acid of the first peptide upon completion of the synthesis or coupling of the full-length amino acid sequence of the first peptide. It is also possible that the second tag is coupled to the N-terminal amino acid of the amino acid sequence of the first peptide and said second tag is coupled, preferably subsequently to the first tag, to the first peptide.

In the following, various embodiments of the first tag will be described in more detail. If not indicated differently, the various embodiments described for the first tag may also be embodiments of the second tag. If the description of features and embodiments is simply referring to a or the tag, this means that such description of features and embodiments is a description of features and embodiments of both the first tag and the second tag.

In an embodiment the tag is selected from the group comprising a peptide, a protease substrate and a leaving group, wherein the leaving group is preferably selected from the group comprising peptides, aromatic amines, phenols or thiophenols, aliphatic amines, aliphatic alcohols or aliphatic thiols.

In the embodiment where the tag is a peptide, such peptide consists of 2 to 10 amino acid residues, preferably 2 to 6 amino acid residues, more preferably 2 to 4 amino acid residues and most preferably 3 amino acid residues. As to the chemistry of such amino acids and amino acid residues, respectively, it is to be acknowledged that the individual amino acid may each and independently of the other amino acid residues of the peptide be a biogenic amino acid or a non-biogenic amino acid. Also the individual amino acid may each and independently of the other amino acid residues of the peptide be an L-amino acid or a D-amino acid. It is also within the present invention that also the individual amino acid may each and independently of the other amino acid residues of the peptide be an alpha-amino acid, a beta-amino acid residue, a gamma-amino acid residue or a delta-amino acid residue. In an embodiment, the amino acid sequence of the tag consists of L-amino acid residues. In another embodiment, the amino acid sequence of the tag consists of D-amino acid residues. In an embodiment, the amino acid sequence of the tag consists of L-alpha amino acid residues. In another embodiment, the amino acid sequence of the tag consists of D-alpha amino acid residues.

In an embodiment, the tag is a peptide and wherein at least one of the amino acid residues of the peptide forming the tag comprises an isotope composition which is different from the isotope composition obtained in a non-isotope discriminating method of synthesis of the peptide. As preferably used herein, a non-isotope discriminating method of synthesis of the peptide is a method for the synthesis where the individual isotopes of the atoms and thus the amino acids containing the individual isotopes are not discriminated. As a consequence, the isotopes of the individual atoms are not biased. Such bias can be such that one or several isotopes are enriched. Alternatively, such bias can be such that one or several isotopes is depleted. Finally, the bias can also be such that one species or group of species of isotopes is enriched, whereas another species or group of species of isotopes is depleted. As the isotopes and their distribution play a key role in mass spectrometry used in connection with the method of the invention, the use of a non-isotope discriminating method for the synthesis of the tag is preferred.

In an embodiment, the tag is a protease substrate or part of a protease substrate, whereby the protease substrate is preferably selected from the group comprising peptides, aromatic amides, aromatic esters or thioesters, aliphatic amides, esters or thioesters. In an embodiment, the protease substrate is a peptide, whereby preferably the peptide comprises or consists of 1 to 20 amino acid residues, preferably 1 to 10 amino acid residues and more preferably 1 to 5 amino acid residues and most preferable 1-3 amino acid residues.

In an embodiment, the tag comprises a reactive group, preferably a chemically reactive group. Such reactive group allows the coupling of a label to the tag. In an embodiment, the coupling occurs by the formation of a chemical bond between the tag and the label. In an alternative embodiment, the label comprises a reactive group, preferably a chemically reactive group. Such reactive group allows the coupling of the tag to the label. In an embodiment the coupling occurs by the formation of a chemical bond between the tag and the label.

As to the coupling of the label to the tag, it is within the present invention that the label is coupled to the tag after the coupling of the tag to the first peptide. In an alternative embodiment, the label is coupled to the tag prior to the coupling of the tag to the first peptide.

In an embodiment, of either or both of the first tag and the second tag, the label is part of an amino acid. In accordance therewith, the amino acid is preferably a modified amino acid. Such amino acid, preferably the modified amino acid, is used in the synthesis of the tag. In a preferred embodiment, the tag is a peptide comprising such amino acid. In an embodiment such modified amino acid is meta-nitro-tyrosine as, e.g., described in Cawley et al., J. Biol. Chem. 278, (2003).

The label attached to the tag, either or both the first tag and the second tag, is preferably selected from the group comprising a mass label, a fluorescence label, and a UV label.

In an embodiment, the mass label is contained in a cage compound. Caged compounds are known in the art and, e.g., described in Capello et al., Cancer Biother. Radiopharm. 18, 2003, Storch et al., J. Nucl. Med. 46, 2005.

In the embodiment where the label is a mass label, the mass label is an isotope label, wherein the isotope is preferably selected from the group comprising C isotopes, N isotopes, Cl isotopes and Br isotopes. Preferred C isotopes as ¹³C. Preferred N isotopes are ¹⁵N isotopes. Preferred Br isotopes are ⁷⁹Br and ⁸¹Br. Preferred Cl isotopes are ³⁵Cl and ³⁷Cl.

In the embodiment where the label is a fluorescent label, the fluorescent label is preferably selected from the group comprising β-7-methoxy-coumaryl-alanine, coumaryl derivatives, naphthylamine derivatives, hydroxyl and thiol derivatives of naphthalene, amino-, hydroxyl- or thiol-fluorescein derivatives, amino-, hydroxyl- or thiol-rhodamine derivatives, and aminobenzoic acid derivatives as described, among others, in Handbook of Proteolytic Enzymes, Academic Press, 1998, or in The Handbook - A Guide to Fluorescent Probes and Labeling Technologies, Invitrogen, http://www.invitrogen.com/site/us/en/home/References/Molecular-Probes-The-Handbook.html.

In the embodiment where the label is a UV label, the UV label is preferably selected from the group comprising substituted aromatic amines, amides, phenols, thiophenols, esters, thioesters like substituted anilines, substituted anilides, substituted phenols, substituted thiophenols, substituted naphthylamines, substituted naphthylamides, substituted thioarylesters, substituted arylesters and substituted aliphatic amines, amides, alcohols, thiols, esters, thioesters like substituted primary and secondary amines, substituted secondary and tertiary amides, substituted alcohols, substituted thiols, substituted thioesters, substituted esters as, among others, described in Handbook of Proteolytic Enzymes, Academic Press, 1998, Peters et al., Curr. Microbiol. 18, 1989; Harper et al., Anal. Biochem. 118, 1981; Janowski et al., Anal. Biochem. 252, 1997; Glucksman et al., Biophys. J. 62, 1992, or Soeda et al., Chem. Pharm. Bull. 33, 1985.

It is within the present invention that the tag or a first part of the tag is removable after the coupling to the first peptide, i.e. the tag or a first part thereof is removed, again, from construct comprising the tag or a first part of the tag. The advantage arising from this is that, a stoichiometric relationship between the tag, more specifically the removed tag, and the first peptide existing, the thus removed or released tag is an indication of the first peptide, more specifically the first peptide coupled to the tag. This allows the quantification of the first peptide and the first peptide coupled to the tag, prior to the removal of the tag, respectively, by quantifying the tag. Although both the first tag and the second tag may be used as the tag on which the quantification is based, it is preferred that the tag used for the quantification is the first tag.

The removal of the tag from the first peptide may occur by chemical cleavage, enzymatic cleavage and/or physical cleavage. The means for performing such chemical cleavage, enzymatic cleavage or physical cleavage are known to a person skilled in the art. In a preferred embodiment, such means are those described herein in connection with sequence-specific hydrolysis with which the C-terminal end of a peptide is generated. In an embodiment, the means used in the cleavage of the tag from the first peptide is the one which is used in the sequence-specific hydrolysis with which the C-terminal end of a peptide is generated. In a preferred embodiment, the removal of the first tag or a first part thereof is sequence-specific. This, preferably, follows from the use of said means, i.e. means for chemical cleavage, enzymatic cleavage and physical cleavage which, in such embodiment, are means for sequence-specific chemical cleavage, means for sequence-specific enzymatic cleavage or means for sequence-specific physical cleavage.

One of the functions of the tag in connection with the instant invention is to provide a signal which may be detected. Such signal can be provided by the tag, by the label attached to the tag, or both. Such signal is for use in a method for determining the presence and/or quantity of a target polypeptide of which a first peptide is a proteotypic peptide, in at least one mixture of different polypeptides making use of an internal peptide standard. Preferably, such method is the method for determining the presence and/or quantity of a target polypeptide of which a first peptide is a proteotypic peptide, in at least one mixture of different polypeptides of the invention. In an embodiment, the signal is indicative of the tag and is even more preferably a quantitative signal. In another embodiment, the signal is proportionate to the tag or a part thereof. It is preferred that the signal follows a 1:1 stoichiometry, i.e. one signal unity corresponds to one tag or label. It will be understood by a person skilled in the art that also other stoichiometries may be used in the practicing of the methods of the invention as long as the stoichiometry is known and reproducible in the practicing of the method.

What has been said for the removal of the tag from the first by chemical cleavage, enzymatic cleavage and/or physical cleavage, in its diverse embodiments disclosed herein, applies or is applicable also to the embodiment where only a part of the tag is removed from the construct comprising both the tag and the first peptide. In this embodiment, thus, the tag is removed from the first peptide only in part. Consequently a part of the tag remains coupled to the first peptide, whereas another part of the tag is removed from the construct comprising the tag and the first peptide. A typical example for this embodiment are the so-called FRET systems where one of the two constituents of the FRET system is separated from the other constituent of the FRET system in the course of the cleaving off of a part of the tag from the construct comprising the first peptide and the tag. By such cleaving off, a signal is generated which may be detected and used in connection with the methods of the invention. FRET systems are, for example, described in Yaron et al. 1979, Bratovanova and Petkov 1987, Meldal et al. 1994, Wang et al. 1990, Lee et al., Blood 103, 204.

Depending on the signal, various detection methods may be used. Such detection methods may be selected from the group comprising fluorescence detection, UV detection and mass detection. It is within the skills of a person of the art to determine which detection method is used for which signal and this for which tag and label, respectively.

The tag may be used for the immobilization of the construct comprising the first peptide and the tag. In an embodiment, the tag is immobilized to a surface and subsequently the first peptide is coupled to the tag. In an embodiment, the tag is immobilized to a surface and the amino acids or groups of amino acids forming the amino acid sequence of the first peptide are coupled or attached to the immobilized tag or to the immobilized tag to which already one or several of the amino acids of the amino acid sequence of the first peptide has/have been coupled or attached. In an embodiment thereof, the tag is the first tag. In an alternative embodiment, the tag is the second tag.

In an embodiment where the second tag is a protease substrate, it is preferred that the protease substrate is selected from the group comprising peptides and peptide derivatives mimicking the N-terminal part of endoprotease substrates. In an embodiment where the first tag is a protease substrate, which is preferably attached to the C-terminal and of the first peptide, it is preferred that the protease substrate is selected from the group comprising peptides and peptide derivatives mimicking the C-terminal part of endoprotease substrates.

In an embodiment, the second tag is a peptide. Preferably, such peptide has one or several of the characteristics of the first tag being a peptide. In an embodiment, the peptide being or being part of the second tag, is attached to the N- terminal end, preferably to the N-terminal amino acid residue of the amino acid sequence of the first peptide.

In the embodiment where a first tag and a second tag are coupled to the first peptide, the first tag and the second tag are preferably different from each other. It is within the present invention that the first tag is coupled to the first peptide prior to the coupling of the second tag. It is, however, also within the present invention that the second tag is coupled to the first peptide prior to the coupling of the first tag. This applies equally in case the first and second tag, respectively, are not coupled as a full length molecule, but also in those embodiments where the building blocks of the first tag and second tag are sequentially attached to the first peptide.

In the embodiment where a first tag and a second tag are coupled to the first peptide, in an embodiment, the first tag and the second tag are different. In the embodiment where a first tag and a second tag are coupled to the first peptide and where the first tag and the second tag are different, the label of the first tag is different from the label of the second tag. Alternatively, the label of the first tag and the label of the second tag are identical.

In the embodiment where a first tag and a second tag are coupled to the first peptide, in an embodiment, the first tag and the second tag are identical, but the label of the first tag and the label of the second tag are different.

In an embodiment, the second tag comprises an anchor moiety. The anchor moiety is preferably attached to a linker, whereby the linker is arranged between the anchor moiety and the second tag. Preferably, the anchor moiety is suitable for attaching the second tag and/or the internal peptide standard to a surface which is preferably a solid surface. In an alternative embodiment, the surface is a non-solid surface such as polymers which are soluble but able to shrink and precipitate if the solvents are changed. Such polymers are known to the person skilled in the art (Reactive and Functional Polymers, Volume 44, Issue 1, 14 April 2000, Pages 41-46, The effect of PEG groups on swelling properties of PEG-grafted-polystyrene resins in various solvents, Byeong-Deog Parka and Yoon-Sik Lee; BioPharm International Supplements, Mar 2, 2010, PEG Precipitation: A Powerful Tool for Monoclonal Antibody Purification, Michael Kuczewski, Emily Schirmer, PhD, Blanca Lain, PhD, Gregory Zarbis-Papastoitsis, PhD; Current Opinion in Chemical Biology, Volume 1, Issue 1, June 1997, Pages 107-113, Synthesis on soluble polymers: new reactions and the construction of small molecules, Dennis J Gravert and Kim D Janda).

In an embodiment, such polymers are modified ethylene glycols. The attaching to a surface may be used for purification purposes of the internal peptide standard. In an embodiment, the anchor moiety allows the reversible attachment of the internal peptide standard to a surface. However, it is also within the present invention that the attachment of the anchor moiety and/or if the internal peptide standard is irreversible. In case of the embodiment where the attachment is irreversible, the attachment is a covalent attachment. In a preferred embodiment, the covalent attachment is established by means of a chemoselective reaction of the anchor moiety, the second tag or the first internal peptide standard.

In an embodiment, the anchor moiety is selected from the group comprising biotin, iminobiotin, desthiobiotin, avidin, streptavidin, whereby the surface to which the anchor moiety binds comprises an interaction partner of these anchor moieties which are known in the art to bind to each other. Alternatively, the anchor moiety is a chemical group allowing chemoselective reaction with the appropriate reactive function on the surface. Such chemical groups represent chemical groups which are not found in the first peptide and/or the first tag like maleimido groups; alpha-halo-ketones like bromopyruvic acid or 4-carboxy-alpha-bromo-acetophenone, alpha-isothiocyanato-ketones like 4-carboxy-alpha-isothiocyanato-acetophenon, aldehydes like carboxybenzaldehyde, ketone like levulinic acid, thiosemicarbazides, thioamides like succinic monothioamide, alpha-bromo-carboxylic acids like bromo acetic acid, hydrazines like 4-hydrazinobenzoic acid, O-alkylhydroxylamines like amino-oxy-acetic acid, and hydrazides like glutaric acid monohydrazide.

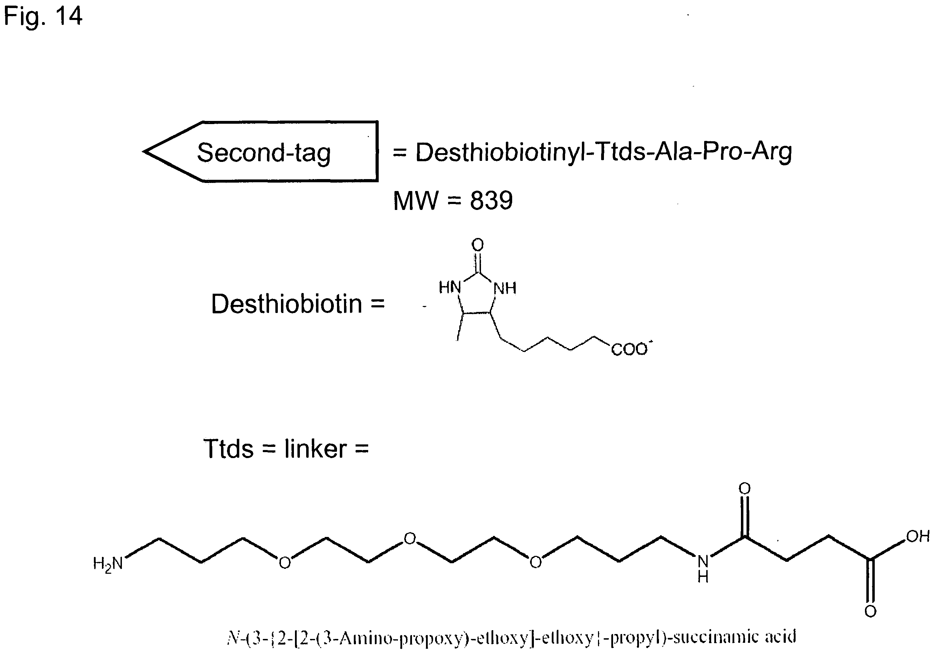

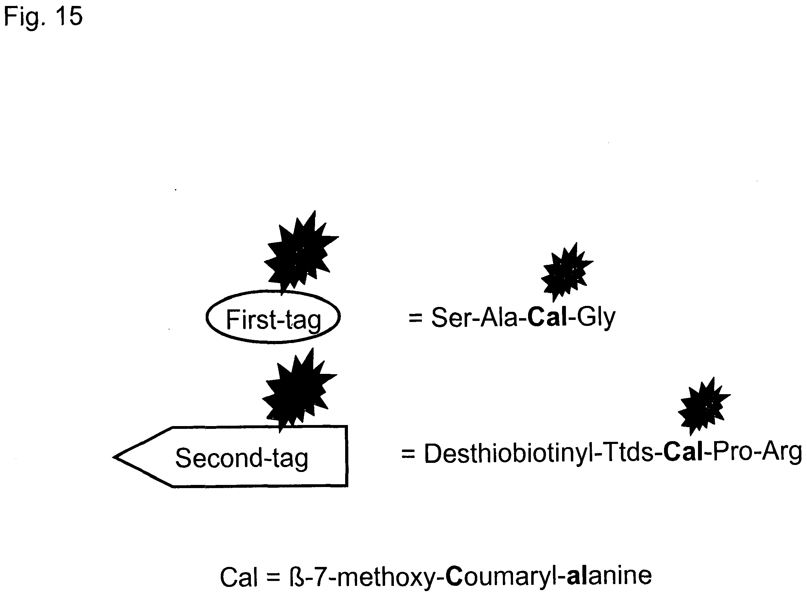

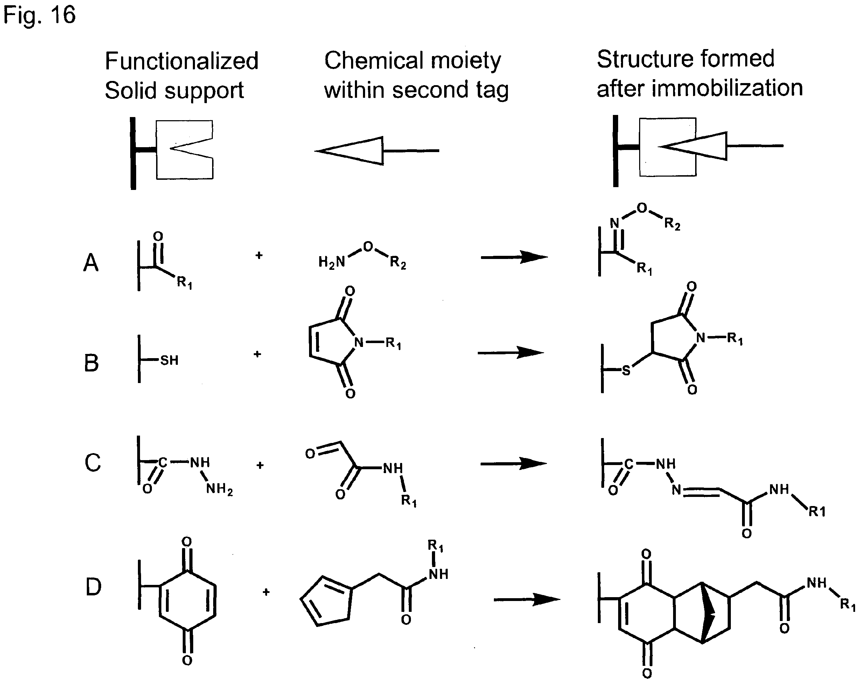

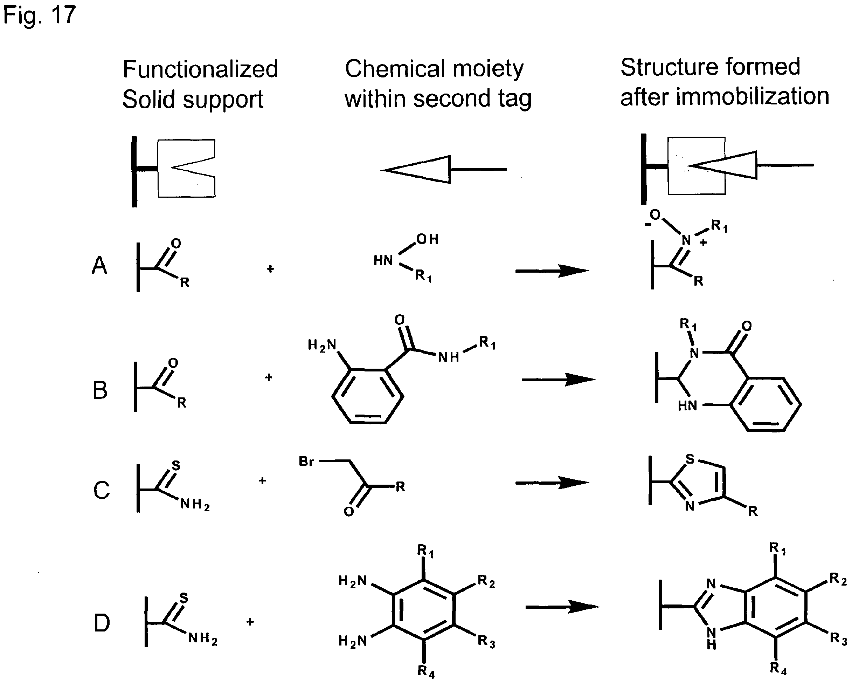

More specifically, a number of reactions known as such to the person skilled in the art can be used so as to effect a chemoselective reaction for the attachment of an anchor moiety or the second tag or the internal standard peptide. Such reaction are described, among others, in Lemieux & Bertozzi (Lemieux, G. A. & Bertozzi, C. R., 1998, Chemoselective ligation reactions with proteins, oligosaccharides and cells, TIBTECH, 16, 506-513, see Figs. 16 and 17 for). With a view to the required directional immobilization it should basically be ensured that under the respective interaction conditions, substantially only one special compound is formed between the amino acid sequence and the surface (Figs. 14 and 15). The choice of the reactive group on the amino acid sequence side will thus depend substantially on the individual sequence. Alternatively it is provided within the scope of the present invention that a terminal structure standard to all the amino acid sequences is provided and this terminal structure is made available for the specific reaction with the surface, especially an activated surface (Figs. 14 and 15). Typically during the chemoselective reactions amino or carboxyl groups contained in the amino acid sequence are not adversely affected. Examples of suitable reactions are the formation of thioethers from halo-carbonic acids and thiols, which include the formation of thioethers from halocarbonic acids and thiols, thioethers from thiols and maleinimides, amide bonds from thioesters and 1,2-aminothiols, thioamide bonds from dithioesters and 1,2-aminothiols, thiazolidines from aldehydes and 1,2-aminothiols, oxazolidines from aldehydes/ketones and 1,2-amino alcohols, imidazoles from aldehydes/ketones and 1,2-diamines (see also Fig. 16 and 17), thiazoles from thioamides and alpha-halo-ketones, aminothiazoles from amino-oxy-compounds and alpha-isothiocyanato-ketones, oximes from amino-oxy-compounds and aldehydes, oximes from amino-oxy-compounds and ketones, hydrazones from hydrazines and aldehydes, hydrazones from hydrazides and ketones.

Moreover, the radicals R₁-R₅ shown in Fig. 16 and 17 or the residues in the above-mentioned chemoselective reactions can be alkyl, alkenyl, alkynyl, cycloalkyl or aryl radicals or heterocyclic compounds, wherein alkyl stands for branched and unbranched C₁₋₂₀-alkyl, C₃₋₂₀-cycloalkyl, preferably for branched and unbranched-C₁₋₁₂ alkyl, C₃₋₁₂₋cycloalkyl, and especially preferably for branched and unbranched C₁₋₆-alkyl, C₃₋₆-cycloalkyl radicals. Alkenyl stands for branched and unbranched C₂₋₂₀-alkenyl, branched and unbranched C₁₋₂₀-alkyl-O-C₂₋₂₀ alkenyl, C₁₋₂₀(-O/S-C₂₋₂₀)₂₋₂₀ alkenyl, aryl-C₂₋₂₀-alkenyl, branched and unbranched heterocyclyl C₂₋₂₀ alkenyl, C₃₋₂₀-cycloalkenyl, preferably for branched and unbranched C₂₋₁₂-alkenyl, branched and unbranched C₁₋₁₂(-O/S-C₂₋₁₂)₂₋₁₂ alkenyl, especially preferably for branched and unbranched C₂₋₆-alkenyl, branched and unbranched C₁₋₆(-O/S-C₂₋₈)₂₋₈ alkenyl radicals; alkynyl stands for branched and unbranched C₂₋₂₀-alkynyl, branched and unbranched C₁₋₂₀(-O/S-C₂₋₂₀)₂-₂₀ alkynyl, preferably for branched and unbranched C₂₋₁₂-alkynyl, branched and unbranched C₁-₁₂(-O/S-C₂₋₁₂)₂₋₁₂ alkynyl, especially preferably for branched and unbranched C₂₋₆-alkynyl, branched and unbranched C₁₋₆(-O/S-C₂₋₈)₂₋₈ alkynyl radicals; cycloalkyl stands for bridged and unbridged C₃₋₄₀-cycloalkyl, preferably for bridged and unbridged C₃₋₂₆-cycloalkyl, especially preferably for bridged and unbridged C₃₋₁₅-cycloalkyl radicals; aryl stands for substituted and unsubstituted mono- or multi-linked phenyl, pentalenyl, azulenyl, anthracenyl, indacenyl, acenaphthyl, fluorenyl, phenalenyl, phenanthrenyl, preferably for substituted and unsubstituted mono- or multi-linked phenyl, pentalenyl, azulenyl, anthracenyl, indenyl, indacenyl, acenaphthyl, fluorenyl, especially preferably for substituted and unsubstituted mono- or multi-linked phenyl, pentalenyl, anthracenyl radicals as well as their partly hydrated derivatives. Heterocyclic compounds can be unsaturated and saturated 3-15-membered mono-, bi- and tricyclic rings with 1-7 heteroatoms, preferably 3-10-membered mono-, bi- and tricyclic rings with 1-5 heteroatoms and especially preferably 5-, 6- and 10-membered mono-, bi- and tricyclic rings with 1-3 heteroatoms.

In addition, at the alkyl, alkenyl, alkynyl, cycloalkyl, aryl, heteroatoms, heterocyclic compounds, biomolecules or natural substance, 0 to 30 (preferably 0 to 10, especially preferably 0 to 5) of the following substituents can occur singly or in combination with one another: fluorine, chlorine, bromine, iodine, hydroxyl, amide, ester, acid, amine, acetal, ketal, thiol, ether, phosphate, sulphate, sulphoxide, peroxide, sulphonic acid, thioether, nitrile, urea, carbamate, wherein the following are preferred: fluorine, chlorine, bromine, hydroxyl, amide, ester, acid, amine, ether, phosphate, sulphate, sulphoxide, thioether, nitrile, urea, carbamate and especially preferred are: chlorine, hydroxyl, amide, ester, acid, ether, nitrile.

In an embodiment, the linker which is arranged between the anchor moiety and the second tag is a chemical structure composed of at least two functional groups. The linker preferably connects the anchor moiety with the first peptide in a covalent manner. The linker spaces the anchor moiety apart from the first peptide allowing the proper interaction of the anchor moiety with the solid surface. Additionally, the linker enables efficient enzymatic cleavage of the second tag/fist peptide-bond.

In an embodiment, the linker is selected from the group comprising N-(3-{2-[2-(3-amino-propoxy)-ethoxy]-ethoxy}-propyl)-succinamic acid or alkanes either branched or linear chains, preferably those composed of 2-30 carbon atoms, more preferably those composed of 4-20 carbon atoms and most preferably those composed of 4-10 carbon atoms; or polyethers, that means polymers of ethylene oxide or propylene oxide, preferably polymers composed of 1-10, more preferably 2-5 ethylene oxide or polypropylene oxide units, or polyalcohols, branched or unbranched, like polyglycol and derivatives thereof like O,O'-bis(2-aminopropyl)-polyethylene glycol or polyurethanes, polyhydroxy acids, polycarbonates, polyimides, polyamides, polyester, polysulfones, preferably those composed of 1-100 monomeric units, more preferably those composed of 1-50 monomeric units and most preferably those composed of 1-20 monomeric units, or combinations of the mentioned alkanes with the mentioned polyurethanes, polyhydroxy acids, polycarbonates, polyimides, polyamides, polyester, polysulfones, or diaminoalkanes either branched or linear chains, preferably those composed of 2-30 carbon atoms, more preferably those composed of 2-20 carbon atoms and most preferably those composed of 2-10 carbon atoms like 1,3-diaminopropane, 1,6-diaminohexane and 1,8-diaminooctane, as well as combinations of diaminoalkanes with polyethers, like 1,4-bis-(3-aminopropoxy)butane or carboxylic acids and dicarboxylic acids and derivatives like hydroxy-, mercapto- and amino carboxylic or dicarboxylic acids with either branched or linear chains, preferably those composed of 2-30 carbon atoms, more preferably those composed of 2-20 carbon atoms and most preferably those composed of 2-10 carbon atoms like succinic acid or glutaric acid. Amino acids and peptides preferably composed of 1-20 residues or more preferably 1-10 residues or most preferably 1-3 residues like trimers of lysine, dimmers of 3-aminopropionic acid and monomers of 6-aminohexanoic acid. Preferably the linker is a combination of a dicarboxylic acid with a diamino polyether like N-(3-{2-[2-(3-amino-propoxy)-ethoxy]-ethoxy} -propyl)-succinamic acid.

In an embodiment, the second tag comprises a peptide comprising an amino acid sequence, whereby the linker is arranged between the anchor moiety and the N-terminal end, preferably between the N-terminal amino acid residue of the second tag.

In an embodiment of the method of the invention, the internal peptide standard comprises a second tag, a first peptide and a first tag, wherein the internal peptide standard is contained in a mixture of peptides, wherein the internal peptide standard is immobilized to a surface, and wherein the internal peptide standard specifically interacts with the surface through the anchor moiety of the internal peptide standard. In one or several subsequent steps, the peptides which are different from the internal peptide standard are removed from the mixture. Preferably, the specificity of the immobilization of the internal peptide standard by means of the second tag and the anchor moiety, respectively, allows for the interaction of the second tag and the anchor moiety, respectively, with the surface. Because of this, any non-binding or non-specifically binding compounds can be removed such as by washing, which results in purification of the intended internal peptide standard, typically immobilized to the surface.

From the above it will be acknowledged that, in an embodiment, the internal peptide standard is attached to a surface. In case the attachment is reversible such as by the use of biotin, desthiobiotin, avidin or streptavidin, the addition of these and similar compounds will result in a competition between the thus immobilized internal peptide standard and the interaction partner of said compounds, whereupon the immobilized internal peptide standard will be released. In an alternative embodiment where the attachment is covalent, the use of a means for sequence-specific cleavage between the second tag and the first peptide, whether or not comprising a first tag, is preferred whereupon such first peptide will be released from the surface. In any case, this kind of procedure provides for a purified internal peptide standard.

The thus obtained or any form of purified internal peptide standard may be further used, preferably in any method described herein, including and in particular the methods for preparing a library of internal peptide standards, wherein the library consists of a plurality of species of an internal peptide standard, and the methods for determining the presence and/or quantity of a target polypeptide of which a first peptide is a proteotypic peptide, in at least one mixture of different polypeptides. For use in said methods, the thus purified internal peptide standard, regardless whether it still contains the second and/or the first tag, is preferably transferred to a different reaction vessel. It will be acknowledged by a person skilled in the art that depending on the various embodiments of said methods, the second tag is removed prior to the used of the internal peptide standard. It will also be acknowledged by a person skilled in the art that depending on the various embodiments of said methods, the first tag is removed prior to the used of the internal peptide standard. Finally, the will be acknowledged by a person skilled in the art that depending on the various embodiments of said methods, the first and the second tag are removed prior to the used of the internal peptide standard. With the removal of the first tag, the second tag or both the first tag and the second tag, there might go along a method for the quantification of the first, second or both the first and the second tag and thus, ultimately, of the internal peptide standard. Methods for the quantification of the first tag, the second tag and the first and the second tag are known to a person skilled in the art. The specific method used depends on the kind of the individual tag and, in the embodiments where the tag bears at least one label, on the kind of label as will be acknowledged by and is evident for a person skilled in the art.

Also described herein is a library or panel of internal peptide standards and a method for preparing the same. Preferably, the library or panel of internal peptide standards comprises a plurality of species of an internal peptide standard. As preferably used herein, a plurality of species of internal peptide standard means at least two internal peptide standards. The method for preparing a library of internal peptide standards may comprise the following steps:
a) individually preparing a plurality of species of an internal peptide standard, whereby each species of an internal peptide standard is prepared according to the method for preparing an internal peptide standard; and
b) optionally mixing the individually prepared species of an internal peptide standard,
wherein the species of an internal peptide standard differ from each other in the first and/or second tag and/or the amino acid sequence of first peptide.

In an embodiment, the first and/or the second tag comprises a peptide having an amino acid sequence and the amino acid sequence of each species of the library of internal peptide standards is different from the amino acid sequence of the other species of the library of internal peptide standards. In an embodiment thereof, the amino acid sequence of the various species differs from each other with regard to the amino acid sequence of the first peptide.

In the embodiment where the amino acid sequence of the various species differs from each other with regard to the amino acid sequence of the first peptide, the first and the second tag of the various species may be the same. If the first and the second tag of the various species are the same and if they bear a label, it is within the invention that the label is the same for the various first and/or second tags of the various species. In such case, the quantification of the label typically provides information on the overall number of internal peptide standard molecules contained in the reaction or vessel, not discriminating between the individual numbers of molecules contributed by the individual various species. If the first and the second tag of the various species are the same and if they bear a label, it is within the invention that the labels of the first tag and/or the second tag of the various species are different; more preferably the label for the first tag of the various species is different and thus characteristic for the individual species. In such case, the quantification of the labels typically provides information on the number of the various individual internal peptide standards contained in the reaction or vessel, discriminating between the individual numbers of molecules contributed by the individual various species. The same considerations apply in case the first tag(s) and the second tag(s) do not require a specific label but that the first tag(s) and the second tag(s) due to their chemical and/or physical characteristics provide the information otherwise conferred by the label. One example for such a case which thus constitutes and embodiment, is the combinatorial use of defined amino acid residues for generation of the sequence(s) of the first and/or the second tag(s) in such a way that the sequence(s) of the first and/or the second tag represent a code for the sequence of the respective first peptide. In other words, the specific first and/or the specific second tag are indicative of a or the specific first peptide and the identification and/or the quantification of the specific first and/or second tag is factually the identification and/or the quantification of the first peptide. Therefore, if there is a second specific first and/or second specific second tag which are indicative of a second specific first peptide, in the method of the invention for determining the presence and/or quantity of a target polypeptide of which a first peptide is a proteotypic peptide, one may discriminate between the said first peptide and said second first peptide in the mixture of different polypeptides, under the proviso that the second first peptide is a proteotypic peptide of a target polypeptide, preferably a second target polypeptide.

The present invention also provides methods for determining the presence and/or quantity of a target polypeptide of which a first peptide is a proteotypic peptide, in at least one mixture of different polypeptides.

In a first basic embodiment, the method for determining the presence and/or quantity of a target polypeptide of which a first peptide is a proteotypic peptide, in at least one mixture of different polypeptides of the invention, is a method for determining the presence and/or quantity of a target polypeptide of which a first peptide is a proteotypic peptide, in at least one mixture of different polypeptides, comprising the following steps:
a) providing a mixture of different polypeptides preferably containing the target polypeptide;
b) adding an internal peptide standard of the invention or of a panel of internal peptide standards of the invention, whereby preferably the amount of the internal peptide standard of the invention or of the panel of internal peptide standards of the invention is unknown, thereby generating a spiked mixture;
c) treating the spiked mixture with a means for sequence-specific hydrolysis to generate a plurality of peptides from the mixture of different polypeptides and to cleave off the first tag and/or the second tag from the internal peptide standard/standards thus releasing the first peptide, whereby the plurality of peptides comprises the proteotypic peptide derived from the different polypeptides and the first peptide released from the internal standard;
d) determining the amount of the first and/or second tag contained in the spiked mixture and therefrom the quantity of the internal standard/standards added in step b);
e) determining the ratio of the first peptide to the proteotypic peptide derived from the different polypeptides, each generated in step c); and
f) calculating from the ratio and the quantity of the internal standard/standards determined in step d) the quantity of the target polypeptide in the mixture of different polypeptides.
It will be acknowledged by a person skilled in the art that a mixture of different polypeptides preferably containing the target polypeptide is added to a quantity of an internal peptide standard of the invention or of a panel of internal peptide standards of the invention, thereby generating a spiked mixture.

In this first basic embodiment, the quantity of the internal peptide standard of the invention is not known when such internal peptide standard is added to the mixture of different polypeptides. The first tag and/or the second tag are only cleaved off from the first peptide when the proteotypic peptide is generated from the mixture of different polypeptides by means of sequence-specific hydrolysis for which the means for sequence-specific hydrolysis as, e.g., disclosed herein, may be used. In this first basic embodiment, it is preferred that the sequence-specific hydrolysis resulting in the release of the proteotypic peptide and the release of either or both of the first and the second tag, preferably the first tag, is effected by the same means for sequence-specific hydrolysis.

In the first basic embodiment, the spiked mixture after having been treated with a means for sequence-specific hydrolysis in step c) may be subjected to a fractionation step. Such fractionation step may be performed by means of a chromatographic procedure, preferably by liquid chromatography. The purpose of such fractionation step is to reduce the complexity of the reaction mixture obtained upon having performed step c). It will be acknowledged by a person skilled in the art that in such step c) a huge number of peptides may be generated by sequence-specific hydrolysis of the mixture of different polypeptides, depending on the number of species of polypeptides contained in said mixture. In complex mixtures such as a biological sample, more specifically a lysate of a cell or organ, the sequence-specific hydrolysis may ultimately result in the generation of hundreds of thousands of peptides. By reducing the complexity of the reaction mixture obtained upon having performed step c) the step of determining the amount of the first and/or second tag contained in the spiked mixture and determining therefrom the quantity of the internal standard/standards added in step b), and more preferably the determining of the ratio of the first peptide to the proteotypic peptide derived from the different polypeptides as subject to step e) can be performed more easily and the performance of the analytical device can be less advanced.

In an embodiment of the method according to the first basic embodiment, after step c) and prior to step e) the treated spiked mixture, i.e. the reaction mixture obtained after having performed step c), is subjected to a separation step where the first peptide and the proteotypic peptide derived from the different polypeptides are separated from components of the treated spiked mixture. This embodiment is factually a very specific embodiment of the immediately preceding embodiment.

In an embodiment of the method according to the first basic embodiment, after step c) and prior to step d) the treated spiked mixture is subjected to a separation step where the first and the second tag is/are separated from components of the treated spiked mixture. By this separation step, first and/or the second tag are removed from the reaction mixture obtained after having performed step c). Such separation of the first tag and/or the second tag can be advantageous insofar that the detection and quantification of the first and/or second tag is not obstructed or obscured by the other compounds contained in the reaction mixture obtained after having performed step c). This allows for a higher accuracy and/or a less complex analytical method for the detection and quantification of the first and/or the second tag.

In a second basic embodiment, the method for determining the presence and/or quantity of a target polypeptide of which a first peptide is a proteotypic peptide, in at least one mixture of different polypeptides of the invention, is a method for determining the presence and/or quantity of a target polypeptide of which a first peptide is a proteotypic peptide, in at least one mixture of different polypeptides, comprising:
a) providing a mixture of different polypeptides preferably containing the target polypeptide;
b) providing an internal peptide standard of the invention or a panel of internal peptide standards of the invention, whereby preferably the provided amount of the internal peptide standard and/or of the panel of internal peptide standards is unknown, treating the internal peptide standard or the panel of internal peptide standard with a means for sequence-specific hydrolysis to cleave off the first tag and/or the second tag from the internal peptide standard or the panel of internal peptide standards thus releasing the first peptide;
either
ca1) adding the reaction mixture obtained in the performance of step b) to the mixture of different polypeptides of step a) or adding the mixture of different polypeptides of step a) to reaction mixture obtained in the performance of step b) and
ca2) treating the mixture obtained in the performance of step ca1) with a means for sequence-specific hydrolysis to generate a plurality of peptides from the mixture of different polypeptides, whereby the plurality of peptides comprises the proteotypic peptide;
or
cb1) treating the mixture of different polypeptides of step a) with a means for sequence-specific hydrolysis to generate a plurality of peptides from the mixture of different polypeptides, whereby the plurality of peptides comprises the proteotypic peptide; and
cb2) adding the reaction mixture obtained in the performance of step b) to the mixture obtained in the performance of the step cb1) or adding to the mixture obtained in the performance of the step cb1) to the reaction mixture obtained in the performance of step b)
whereby after step ca2) and cb2), respectively, a spiked mixture is obtained;
d) determining the amount of the first and/or second tag contained in the spiked mixture and therefrom the quantity of the internal standard/standards added in step b);
e) determining in the spiked mixture the ratio of the first peptide to the proteotypic peptide derived from the different polypeptides; and
f) calculating from the ratio and the quantity of the internal standard/standards determined in step d) the quantity of the target polypeptide in the mixture of different polypeptides.

In this second basic embodiment, two basic alternatives are possible. In a first alternative, steps ca1) and ca2) are performed. In a second alternative, steps cb1) and cb2) are performed. Irrespective thereof, in this second embodiment the treatment of the internal peptide standard(s) with a means for sequence-specific hydrolysis is effected separately from the treatment of the mixture of different polypeptides preferably containing the target polypeptide.

In the second basic embodiment, the reaction mixture obtained after the performing of step ca2), or after the performing of step cb1) or cb2) may be subjected to a fractionation step. Such fractionation step may be performed by means of a chromatographic procedure, preferably by liquid chromatography. The purpose of such fractionation step is to reduce the complexity of the reaction mixture obtained upon having performed step ca2), or the reaction mixture obtained after having performed step cb1) or cb2). It will be acknowledged by a person skilled in the art that in said steps ca2), cb1) and cb2) a huge number of peptides may be generated by sequence-specific hydrolysis of the mixture of different polypeptides, depending on the number of species of polypeptides contained in said mixture. In complex mixtures such as a biological sample, more specifically a lysate of a cell or organ, the sequence-specific hydrolysis may ultimately result in the generation of hundreds of thousands of peptides. By reducing the complexity of the reaction mixture obtained upon having performed steps ca2), cb1) and cb2), respectively, the step of determining the amount of the first and/or second tag contained in the spiked mixture and determining therefrom the quantity of the added internal standard/standards, and more preferably the determining of the ratio of the first peptide to the proteotypic peptide derived from the different polypeptides as subject to step e) can be performed more easily and the performance of the analytical device can be less advanced. In connection with this embodiment, the spiked mixture is also referring to the reaction mixture obtained after the performing of steps ca2) and cb2) and which has been subjected to said fractionation step.

In an embodiment of the method according to the second basic embodiment, prior to step e) the treated spiked mixture, i.e. the reaction mixture obtained after having performed step ca2), cb1) or cb2) is subjected to a separation step where the first peptide and the proteotypic peptide derived from the different polypeptides are separated from components of the treated spiked mixture. This embodiment is factually a very specific embodiment of the immediately preceding embodiment. In connection with this embodiment, the spiked mixture is also referring to the reaction mixture obtained after the performing of steps ca2) and cb2) and which has been subjected to said separation step.

In an embodiment of the method according to the second basic embodiment, prior to step d) the treated spiked mixture is subjected to a separation step where the first and the second tag is/are separated from components of the treated spiked mixture. By this separation step, first and/or the second tag are removed from the reaction mixture obtained after having performed step c). Such separation of the first tag and/or the second tag can be advantageous insofar that the detection and quantification of the first and/or second tag is not obstructed or obscured by the other compounds contained in the reaction mixture obtained after having performed steps ca2), cb1) an dcb2). This allows for a higher accuracy and/or a less complex analytical method for the detection and quantification of the first and/or the second tag. In connection with this embodiment, the spiked mixture is also referring to the reaction mixture obtained after the performing of steps ca2) and cb2) and which has been subjected to said separation step.

In an embodiment of the method according to the second basic embodiment, the means for sequence-specific hydrolysis used in step b) is removed from the mixture obtained in the performance of step b) prior to performing step ca1) or cb1). Such means are known to a person skilled in the art. Non-limiting examples for procedures for the removal of the means for sequence-specific hydrolysis are liquid chromatography, high performance reverse phase liquid chromatography, size exclusion chromatography, electrophoresis, capillary electrophoresis, gel electrophoresis and affinity chromatography. As used in this embodiment, the removal of the means for sequence-specific hydrolysis also encompasses the inactivation of such means. For example, if the means is an enzyme the enzyme may be inactivated by heat treatment, by pH shift or addition of an inhibitor to the enzyme activity.

In a third basic embodiment, the method for determining the presence and/or quantity of a target polypeptide of which a first peptide is a proteotypic peptide, in at least one mixture of different polypeptides of the invention, is a method for determining the presence and/or quantity of a target polypeptide of which a first peptide is a proteotypic peptide, in at least one mixture of different polypeptides, comprising:
a) providing an internal peptide standard of the invention or a panel of internal peptide standards of the invention, whereby the amount of the internal peptide standard of the invention or of the panel of internal peptide standards of the invention is unknown, treating the internal peptide standard or the panel of internal peptide standards with a means for sequence-specific hydrolysis to cleave off the first tag and/or the second tag from the internal peptide standard or the panel of internal peptide standards thus releasing the first peptide;
b) optionally removing the means for sequence-specific hydrolysis;
c) determining the amount of the first and/or second tag contained in the mixture obtained from the performance of step a) of b) and therefrom the amount of the internal standard contained in said mixture;
d) providing a mixture of different polypeptides preferably containing the target polypeptide;
either
ea1) adding a part of the reaction mixture obtained in the performance of step a) or b) to the mixture of different polypeptides of step d) or adding the mixture of different polypeptides of step d) to a part of the reaction mixture obtained in the performance of step a) or b) and
ea2) treating the mixture obtained in the performance of step ea1) with a means for sequence-specific hydrolysis to generate a plurality of peptides from the mixture of different polypeptides, whereby the plurality of peptides comprises the proteotypic peptide;
or
eb1) treating the mixture of different polypeptides of step d) with a means for sequence-specific hydrolysis to generate a plurality of peptides from the mixture of different polypeptides, whereby the plurality of peptides comprises the proteotypic peptide; and
eb2) adding a part of the mixture obtained in the performance of step a) or b) to the mixture obtained in the performance of the step eb1) or adding the mixture obtained in the performance of the step eb1) to adding a part of the mixture obtained in the performance of step a) or b) ;
whereby after step ea2) and eb2), respectively, a spiked mixture is obtained;
f) determining in the spiked mixture the ratio of the first peptide to the proteotypic peptide derived from the different polypeptides; and
g) calculating from the ratio and the quantity of the internal standard/standards the quantity of the target polypeptide in the mixture of different polypeptides.

In this third basic embodiment two basic alternatives are possible. In a first alternative, steps ea1) and ea2) are performed. In a second alternative, steps eb1) and eb2) are performed. Irrespective thereof, in this second embodiment the treatment of the internal peptide standard(s) with a means for sequence-specific hydrolysis is effected separately from the treatment of the mixture of different polypeptides preferably containing the target polypeptide and, additionally, as step c), the amount of the first and/or second tag contained in the mixture obtained from the performance of step a) of b) is determined and, therefrom, the amount of the internal standard contained in said mixture is determined. In accordance therewith, with the adding of a part of the reaction the reaction mixture obtained in the performance of step a) or b), preferably by means of a known aliquot of said reaction mixture, a known amount of the internal peptide standard is added to the mixture of different polypeptides preferably containing the target polypeptide, regardless whether such mixture of different polypeptides preferably containing the target polypeptide has at that stage of the method been subject to sequence-specific hydrolysis.

In the third basic embodiment, the reaction mixture obtained after the performing of step ea2), or after the performing of step eb1) or eb2) may be subjected to a fractionation step. Such fractionation step may be performed by means of a chromatographic procedure, preferably by liquid chromatography. The purpose of such fractionation step is to reduce the complexity of the reaction mixture obtained upon having performed step ea2), or the reaction mixture obtained after having performed step eb1) or eb2). It will be acknowledged by a person skilled in the art that in said steps ea2), eb1) and eb2) a huge number of peptides may be generated by sequence-specific hydrolysis of the mixture of different polypeptides, depending on the number of species of polypeptides contained in said mixture. In complex mixtures such as a biological sample, more specifically a lysate of a cell or organ, the sequence-specific hydrolysis may ultimately result in the generation of hundreds of thousands of peptides. By reducing the complexity of the reaction mixture obtained upon having performed steps ea2), eb1) and eb2), respectively, the step of determining of the ratio of the first peptide to the proteotypic peptide derived from the different polypeptides as subject to step f) can be performed more easily and the performance of the analytical device can be less advanced. Particularly, in case the ratio of the first peptide to the proteotypic peptide derived from the different polypeptides is determined by mass spectrometry, a mass spectrometer can be used having a lower resolution which allows that the methods of the invention can be used also by laboratory not having the most sophisticated mass spectrometers at their disposal. In connection with this embodiment, the spiked mixture is also referring to the reaction mixture which has been subjected to said fractionation step and which is obtained after the performing of steps ea2) and eb2).

In an embodiment of the method according to the third basic embodiment prior to step f) the treated spiked mixture, i.e. the reaction mixture obtained after having performed step ea2), eb1) or eb2) is subjected to a separation step where the first peptide and the proteotypic peptide derived from the different polypeptides are separated from components of the treated spiked mixture. This embodiment is factually a very specific embodiment of the immediately preceding embodiment. In connection with this embodiment, the spiked mixture is also referring to the reaction mixture which has been subject to said fractionation step and which is obtained after the performing of steps ea2) and eb2).

In an embodiment of the method according to the third embodiment prior to step ea1) the reaction mixture obtained in the performance of step a) or b) is subjected to a separation step where the first and the second tag is/are separated from components of said reaction mixture. Such separation of the first tag and/or the second tag can be advantageous insofar that the detection and quantification of the first and/or second tag is not obstructed or obscured by the other compounds contained in the reaction mixture obtained after having performed steps ea2), eb1) and eb2). This allows for a higher accuracy and/or a less complex analytical method for the detection and quantification of the first and/or the second tag. In connection with this embodiment, the spiked mixture is also referring to the reaction mixture obtained after the performing of steps ea2), eb1) and eb2) and which has been subjected to said separation step. In an alternative embodiment of the method according to the third embodiment, prior to step eb2) the reaction mixture obtained in the performance of step eb1) is subjected to a separation step where the proteotypic peptide derived from the different polypeptides is separated from components of said reaction mixture. This embodiment is factually a very specific embodiment of the above described embodiment comprising the fractionation step. In connection with this embodiment, the spiked mixture is also referring to the reaction mixture obtained after the performing of steps ea2), eb1) and eb2) and which has been subjected to said separation and/or fractionation step.

In an embodiment of the method according to the third embodiment, the means for sequence-specific hydrolysis used in step a) is removed from the mixture obtained in the performance of step a) prior to performing step c) or d). In a further embodiment of the method according to the third basic embodiment, the means for sequence-specific hydrolysis upon having been used in steps ea1) or in step eb1) is removed prior to performing step f). Such means are known to a person skilled in the art. Non-limiting examples for procedures for the removal of the means for sequence-specific hydrolysis are liquid chromatography, high performance reverse phase liquid chromatography, size exclusion chromatography, electrophoresis, capillary electrophoresis, gel electrophoresis and affinity chromatography. As used in this embodiment, the removal of the means for sequence-specific hydrolysis also encompasses the inactivation of such means. For example, if the means is an enzyme the enzyme may be inactivated by heat treatment or addition of an inhibitor to the enzyme activity.

In each and any of the first, second and third basic embodiment of the method of the invention for determining the presence and/or quantity of a target polypeptide of which a first peptide is a proteotypic peptide, in at least one mixture of different polypeptides, the first peptide of the internal peptide standard/standards comprise at least one mass label. The use of the mass label allows determining the ratio of the first peptide to the proteotypic peptide derived from the different polypeptides.

In connection with the methods of the invention for determining the presence and/or quantity of a target polypeptide of which a first peptide is a proteotypic peptide, in at least one mixture of different polypeptides, the ratio of the first peptide released from the internal peptide standard to the proteotypic peptide derived from the different polypeptides is preferably determined by mass spectrometry.

In each and any of the first, second and third basic embodiment of the method of the invention for determining the presence and/or quantity of a target polypeptide of which a first peptide is a proteotypic peptide, in at least one mixture of different polypeptides, the mass label is preferably selected from the group comprising C isotopes and N isotopes. Suitable C isotopes and N isotopes are known to the person skilled in the art. Preferred C isotopes are those which allow distinction of the first tag and/or the second tag and/or the first peptide and/or the proteotypic peptide, preferably the distinction of the first peptide from the proteotypic peptide, by mass spectrometry. Such preferred C isotopes are preferably selected from the group comprising ¹³C isotopes. Preferred N isotopes are those which allow distinction of the first tag and/or the second tag and/or the first peptide and/or the proteotypic peptide, preferably the distinction of the first peptide from the proteotypic peptide, by mass spectrometry. Such preferred N isotopes are preferably selected from the group comprising ¹⁵N isotopes. In this embodiment, the internal peptide standard may contain a heavier or lighter isotope compared to the isotope of the mixture of different polypeptides preferably containing the target polypeptide and the proteotypic peptide, respectively. However, it is also within the present invention that the mixture of different polypeptides preferably containing the target polypeptide and the proteotypic peptide, respectively, may contain a heavier or lighter isotope compared to the isotope contained in the internal peptide standard. Such isotopes can be incorporated into the internal peptide standard by the use of building blocks of such internal peptide standard which contains the respective isotope(s), and/or such isotopes can be incorporated into the different polypeptides of the mixture of different polypeptides preferably containing the target polypeptide and the proteotypic peptide, respectively, by the use of building blocks of such different polypeptides and the proteotypic peptide bearing such isotope(s). In case the different polypeptides or the mixture of different polypeptides is derived from a biological system such as a cell, tissue, organ or organism the respectively labelled different polypeptides and proteotypic peptide can be obtained by feeding respectively labelled building blocks or precursors of such different polypeptides and proteotypic peptide to such biological system.

Due to this difference in molecular mass arising from the use of differently labelled first peptide and proteotypic peptide, it is possible to determine the ratio of the first peptide to the proteotypic peptide derived from the different polypeptides, preferably by mass spectrometry.

Mass spectrometry as can be used in each and any of the first, second and third basic embodiment of the method of the invention for determining the presence and/or quantity of a target polypeptide of which a first peptide is a proteotypic peptide, in at least one mixture of different polypeptides, is known to a person skilled in the art and, for example, described in Gsteiger and Aebersold, Nat. Rev. Genet. 10, 2009 In a preferred embodiment, the mass spectrometry is multistage mass spectrometry. Pan et al., J. Proteome Res. 8, 2009.

In each and any of the first, second and third basic embodiment of the method of the invention for determining the presence and/or quantity of a target polypeptide of which a first peptide is a proteotypic peptide, in at least one mixture of different polypeptides, a peptide signature obtained by fragmentation which is diagnostic for the presence of the peptide is preferably known for the proteotypic peptide and thus for the first peptide, too. In an embodiment thereof, the peptide signature obtained by fragmentation is subjected to subsequent analysis, preferably in a tandem mass spectrometer. Methods for the fragmentation are known to a person skilled in the art and are selected from the group comprising collision induced dissociation (see, e.g., Wells JM, McLuckey SA (2005). "Collision-induced dissociation (CID) of peptides and proteins". Meth. Enzymol. 402: 148-85), electron capture dissociation (see, e.g., Zubarev RA, Kelleher NL, McLafferty FW (1998). "Electron capture dissociation of multiply charged protein cations. A nonergodic process". J. Am. Chem. Soc. 120 (13): 3265-66.), electron transfer dissociation (see, e.g., Syka JE, Coon JJ, Schroeder MJ, Shabanowitz J, Hunt DF (2004). "Peptide and protein sequence analysis by electron transfer dissociation mass spectrometry". Proc. Natl. Acad. Sci. U.S.A. 101 (26): 9528-33), infrared multiphoton dissociation (see, e.g., Little DP, Speir JP, Senko MW, O'Connor PB, McLafferty FW (1994). "Infrared multiphoton dissociation of large multiply charged ions for biomolecule sequencing". Anal. Chem. 66 (18): 2809-15), and blackbody infrared radiative dissociation (see, e.g., Schnier PD, Price WD, Jockusch RA, Williams ER (1996). "Blackbody Infrared Radiative Dissociation of Bradykinin and Its Analogues: Energetics, Dynamics, and Evidence for Salt-Bridge Structures in the Gas Phase". Journal of the American Chemical Society 118 (30): 7178-7189).

In each and any of the first, second and third basic embodiment of the method of the invention for determining the presence and/or quantity of a target polypeptide of which a first peptide is a proteotypic peptide, in at least one mixture of different polypeptides, the internal peptide standard of the invention or the panel of internal standards of the invention may be purified prior to the use in said method of the invention for determining the presence and/or quantity of a target polypeptide of which a first peptide is a proteotypic peptide, in at least one mixture of different polypeptides. In a preferred embodiment, the purification is based on or makes use of the second tag. In such embodiment, the by-products of the synthesis of the internal peptide standard or of the panel of internal peptide standards are preferably removed from the reaction mixture while or with the internal peptide standard or the panel of internal peptide standard being immobilized or attached to a surface as also disclosed herein. In an alternative embodiment, the internal peptide standard of the invention or the panel of internal peptide standards of the invention is not purified prior to the use in said method of the invention for determining the presence and/or quantity of a target polypeptide of which a first peptide is a proteotypic peptide, in at least one mixture of different polypeptides. In such alternative embodiment, the internal peptide standard of the invention or the panel of internal peptide standards of the invention may be used as obtained from the method of the invention for preparing an internal peptide standard.

In each and any of the first, second and third basic embodiment of the method of the invention for determining the presence and/or quantity of a target polypeptide of which a first peptide is a proteotypic peptide, in at least one mixture of different polypeptides, the amount of the internal peptide standard or of the panel of internal peptide standards is preferably determined based on the amount of the first tag or the second tag, preferably the first tag, which is attached to said internal peptide standard or internal peptide standards. In an embodiment thereof, the tag comprises a fluorescence label known to a person skilled in the art and of which some non-limiting examples are disclosed herein. The amount of the tag is determined by measuring the fluorescence signal of the label. In an alternative embodiment, the tag comprises a UV label known to a person skilled in the art and of which some non-limiting examples are disclosed herein. The amount of the tag is determined by measuring the UV signal of the label.

In each and any of the first, second and third basic embodiment of the method of the invention for determining the presence and/or quantity of a target polypeptide of which a first peptide is a proteotypic peptide, in at least one mixture of different polypeptides, preferably the mixture of different polypeptides is preferably a sample. In an embodiment, such sample contains at least one polypeptide, preferably more than one polypeptide. The sample may be a biological sample or a non-biological sample. A biological sample may be a sample obtained from biological material. The biological material may be selected from the group comprising a body fluid sample, a lysate or preparation of a cell or a part thereof, a lysate or preparation of a tissue or a part thereof, a lysate or preparation of an organ or part thereof, a lysate or preparation of a complete organism or part thereof, a fermentation broth or part thereof and a cell culture fluid or part thereof. The body fluid sample may be a sample of a body fluid, whereby the body fluid is selected from the group comprising blood, plasma, serum, urine, liquor, sputum, faeces. The non-biological sample may be a sample from a chemical reaction. The chemical reaction may be a synthesis of polypeptides or a process of chemical modification of polypeptides simulating posttranslational modifications.

To the extent it is indicated in the methods of the invention that the mixture of different polypeptides preferably contains a or the target polypeptide, preferably, this is to mean that the mixture either contains said target polypeptide or does not contain said target polypeptide. In a preferred embodiment, the mixture contains said target polypeptide. It is obvious to a person skilled in the art that if the mixture does not contain said target polypeptide, its amount cannot be determined. Nevertheless, the finding when performing the methods of the invention for determining the presence and/or quantity of a target polypeptide of which a first peptide is a proteotypic peptide, in at least one mixture of different polypeptides, that the mixture does not contain said target polypeptide, may already constitute a valuable piece of information and a valuable result obtained by said methods.

To the extent it is indicated in the methods of the invention that the amount of the internal peptide standard of the invention or of the panel of internal peptide standards of the invention which is added to a compound, vessel reaction mixture or something similar, is unknown, preferably, this is to mean that the number of molecules of the internal peptide standard of the invention or of the panel of internal peptide standards of the invention is unknown which is added to said compound, vessel, reaction mixture or something similar. In connection therewith, it is to be acknowledged that the adding of the internal peptide standard of the invention or of the panel of internal peptide standards of the invention preferably occurs by transferring an aliquot of a liquid containing said internal peptide standard of the invention or of the panel of internal peptide standards of the invention. Preferably, the volume of the aliquot is known in the performing of the method of the invention. However, it is also within the present invention that the volume of the aliquot is not known. Particularly in the latter embodiment, however, it is preferred that the volume of the reaction mixture which is obtained by adding said internal peptide standard of the invention or of the panel of internal peptide standards of the invention to said compound, vessel, reaction mixture or something similar, is known.

As preferably used, any wording which specifies the limits of a range such as, e. g., "from 1 to 5" means any integer from 1 to 5, i.e. 1, 2, 3, 4 and 5. In other words, any range that is defined by two integers comprises both the two integers defining said limits of the definition and any integer comprised by or contained within said range.

Further features, embodiments and advantages of the present invention may be taken from the following figures and examples, whereby
Fig. 1 is a schematic representation illustrating the generation of an internal peptide standard of the invention;
Fig. 2 is a schematic representation illustrating the quantification of a purified internal peptide standard of the invention;
Fig. 3 is a schematic representation illustrating the quantification of a non-purified internal peptide standard of the invention which is synthesized with capping steps;
Fig. 4 is a schematic representation illustrating the quantification of a non-purified internal peptide standard analysed by HPLC-MS;
Fig. 5 is a schematic representation illustrating the experiment showing the compatibility of chromophore incorporated into first tag with enzymatic cleavage of the internal peptide standard;
Fig. 6 is a chromatogram indicating the run characteristics of a first peptide labelled with a first tag prior to and subsequent to tryptic digest as discussed in Example 2;
Fig. 7 shows the composition of a first tag containing meta-nitro-tyrosine;
Fig. 8 shows UV-Vis spectra of acetylated first tag containing meta-nitro-tyrosine as discussed in Example 3;
Fig. 9 shows the result of a quantification of non-purified internal peptide standard analysed by HPLC-ESI-MS equipped with a UV-Vis detector;
Fig. 10 is a schematic representation illustrating the generation and use of an internal peptide standard comprising both a first and a second tag;
Fig. 11 is a schematic representation illustrating off-support cleavage of an internal peptide standard;
Fig. 12 is a schematic representation illustrating on-support cleavage of an internal peptide standard;
Fig. 13 is a schematic representation illustrating various options for quantifying first peptides released by cleavage of purified internal peptide standards;
Fig. 14 shows the composition of a second tag containing desthiobiotin;
Fig. 15 shows the composition of a first and second tag containing fluorescence label;
Figs. 16 and 17 indicate various chemoselective chemical reactions which can be used for immobilization of peptide derivatives onto supports;
Fig. 18 is a diagram indicating absorption units as a function of the concentration of the first tag illustrated in Fig. 7 whereby absorption is determined at either 350 nm or 410 nm; and
Fig. 19 shows straight calibration lines for the quantification of five different labeled peptides and the standard Ac-SA-nitroTyr-R-OH.

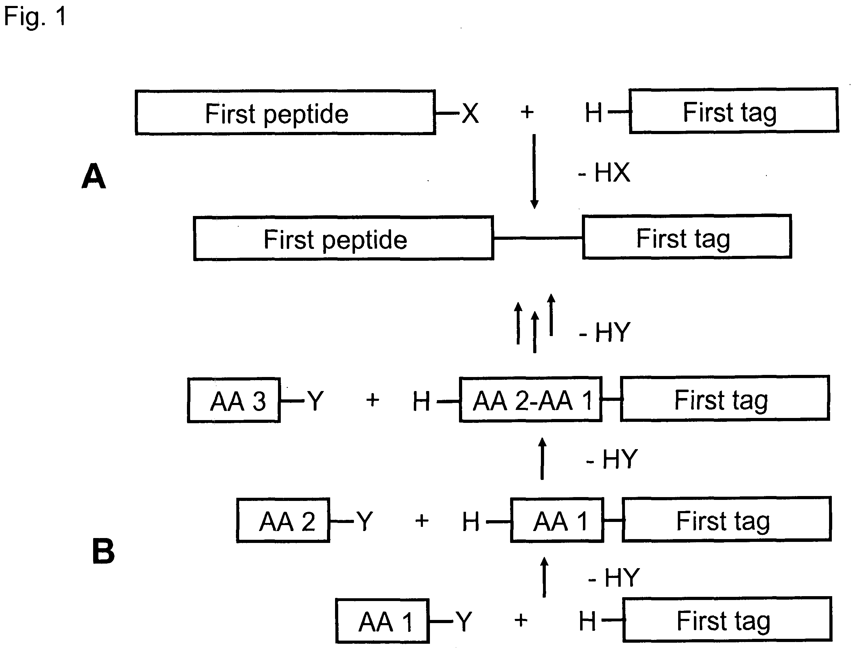

Fig. 1 is a schematic representation for the generation of an internal peptide standard of the invention. In Fig. 1 A) a) pre-synthesized first tag is fused to the pre-synthesized first peptide in one step yielding the internal peptide standard. As illustrated in Fig. 1 B) if the first tag is synthesized, in a step by step manner, additional amino acids are added in a stepwise manner according to peptide synthesis protocols of the state of the art such as described in EP 0 651 762. After the coupling of the last amino acid residue the amino acid sequence of the first peptide is completed and subsequent to removal of protecting groups and optional removal of the compound from a solid synthesis support the synthesis of the internal peptide standard is completed.

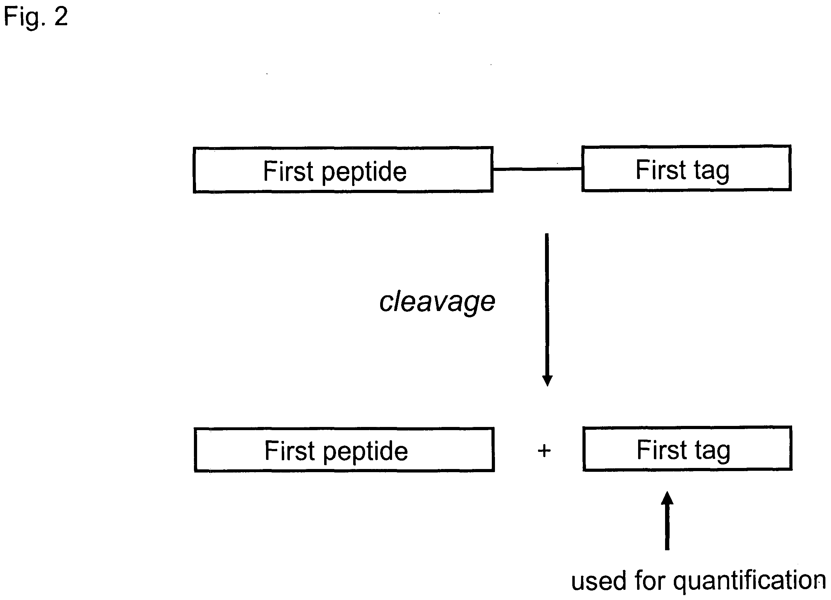

Fig. 2 is a schematic representation illustrating the quantification of a purified internal peptide standard of the invention. The purified internal peptide standard, ideally 100% pure or at least 98% pure or 95% pure, is cleaved with a means for sequence-specific cleavage to release the fist tag and the first peptide in a defined ratio. Preferably, the ratio is 1:1. If the cleavage reaction is nearly 100 % and if the starting purity is high the amount of the released first tag is proportional to the amount of the released first peptide.

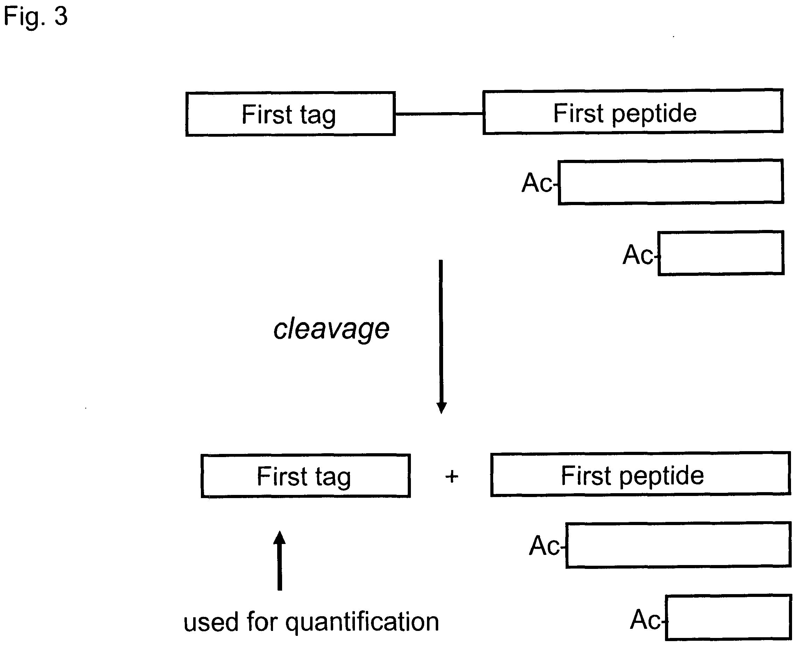

Fig. 3 is a schematic representation illustrating the quantification of a non-purified internal peptide standard of the invention which is synthesized with capping steps. In this embodiment, the internal peptide standard is synthesized by a modified procedure. After each coupling step and before each removal of the protecting group there is an additional capping step introduced. If, e.g., appropriately activated acetic acid is used, the side products resulting from incomplete coupling steps are acetylated and thereby no longer chemically reactive. At the end of the synthesis of the first peptide the first tag is added. Because of the capping steps the first tag will react with the first peptide only and not with acetylated impurities. At the end of the synthesis the desired internal peptide standard is created together with acetylated impurities which do not carry the first tag. Subsequent to a cleavage reaction the first tag could be released from the first peptide only. Therefore, in this case the amount of released first tag is indicative for the amount of released first peptide.

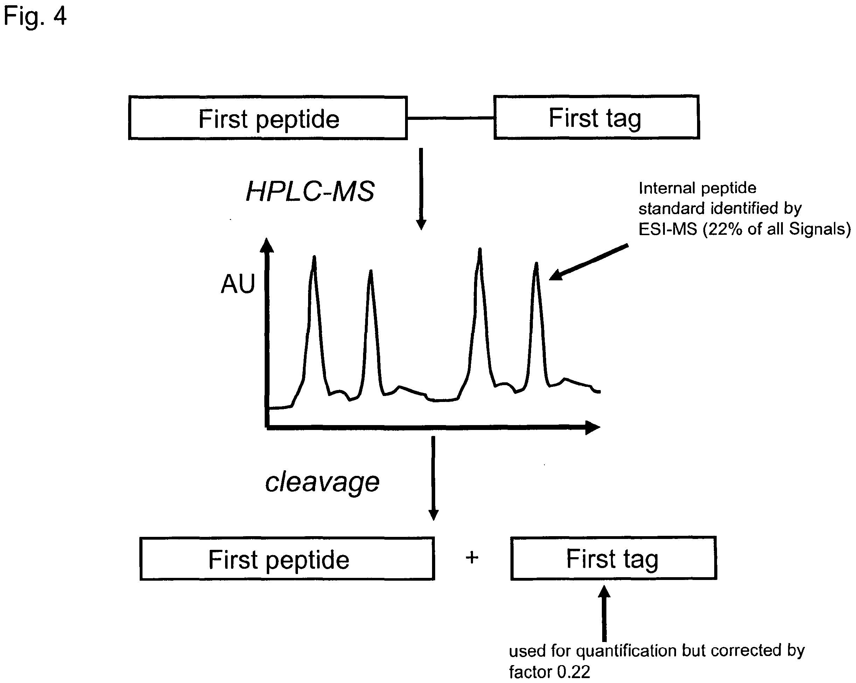

Fig. 4 is a schematic representation illustrating the quantification of a non-purified internal peptide standard analysed by HPLC-MS. The internal peptide standard is synthesized and analysed by HPLC coupled to a mass spectrometer equipped with an UV-Vis detector. During the HPLC run the internal peptide standard is separated from the impurities which still carry the first tag. The signal for the internal peptide standard can be identified by the mass spectrometer coupled to the HPLC. The relative amount of first tag bound to the first peptide could be calculated from the UV-Vis trace of the HPLC run. This calculation yields the relative amount of internal standard in percent within the non-purified internal peptide standard. Subsequent to an appropriate cleavage reaction the amount of the released first tag is measured and then corrected for the relative content of the starting material. In this example the UV-Vis trace yielded 22% of all first tag containing peptides which are corresponding to the internal peptide standard which was identified by the mass trace. Subsequent to cleavage the measured total amount of released first tag has to be corrected by a factor of 0.22 reflecting the 22% content of the internal peptide standard.

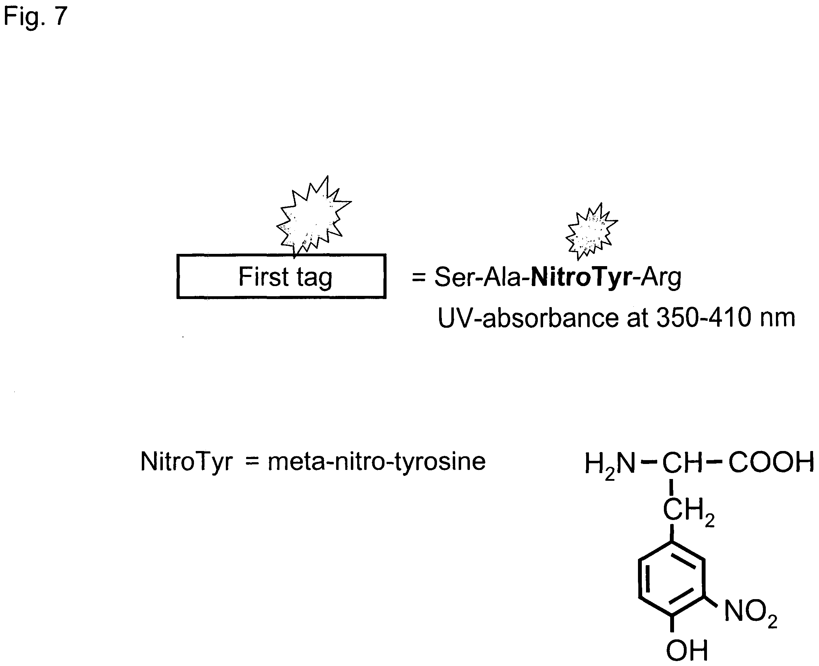

Fig. 7 indicates the composition of a first tag containing meta-nitro-tyrosine. The composition of a fist tag is shown using the one letter code for the naturally occurring amino acid residues. This compound is a representative example of a first tag optimized for cleavage using protease trypsin. Serine and alanine residues are occupying the P1' and P2'-position, respectively. The chromophore-containing amino acid residue meta-nitro-tyrosine is optimized for the P3'-position. In the P4'-position an arginine residue is used. The structure of the modified amino acid residue meta-nitro-tyrosine is given. Depending on the protonation state of the hydroxyl group of this residue the aromatic ring systems have a strong absorbance with a maximum at 350 nm for the protonated state of the hydroxyl group and a maximum at 410 nm for the dissociated form of the hydroxyl group.

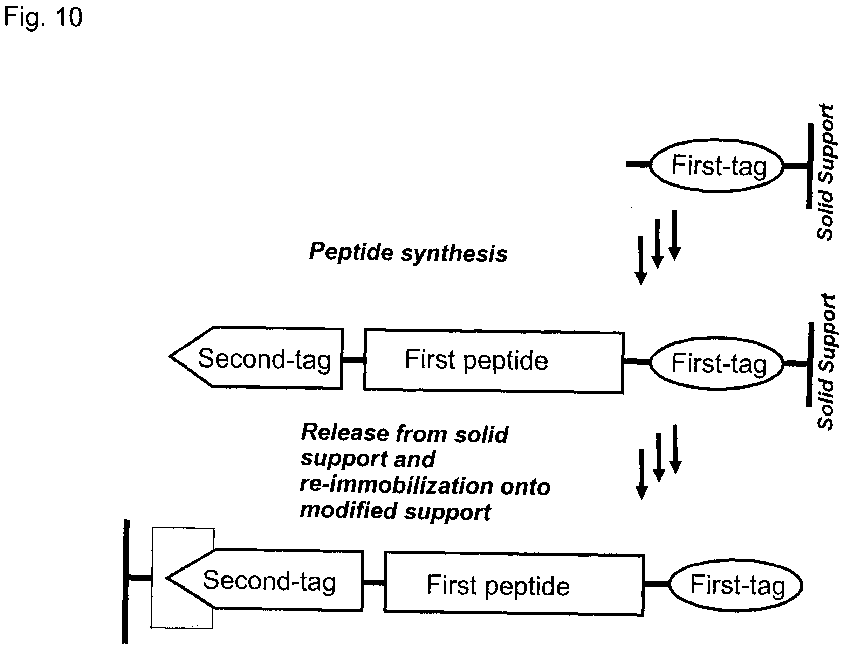

Fig. 10 is a schematic representation illustrating the generation and use of an internal peptide standard comprising both a first tag and a second tag. More complex peptide standards can be produced using peptide synthesis as illustrated in Fig. 10. Subsequent to generation of the first tag on a solid support, either by stepwise synthesis or by attachment of a pre-synthesized tag directly to the solid support, a first peptide followed by a second tag are synthesized in a stepwise manner. Alternatively, the first peptide is attached to the first tag and then the second tag is attached to the bound first-peptide-first-tag structure. Additionally, combinations of stepwise synthesis and attachment of pre-synthesized structures are possible.

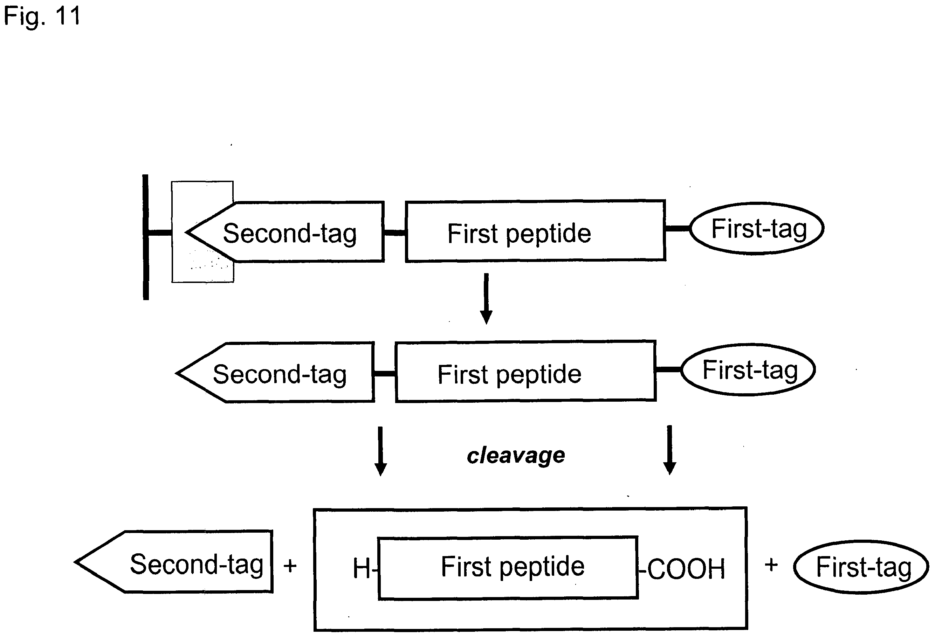

Fig. 11 is a schematic representation illustrating off-support cleavage of an internal peptide standard comprising both a first tag and a second tag. The internal peptide standard immobilized to a support via the second tag could be released from that support subsequent to washing steps. These washing steps will remove side products of peptide synthesis which did not bind specifically or which did not form a covalent bond. The released, and purified, internal peptide standard could be cleaved yielding the first peptide together with the second tag and the first tag. The first tag and/or the second tag can be used for quantification of the amount of the first peptide generated by the cleavage reaction.

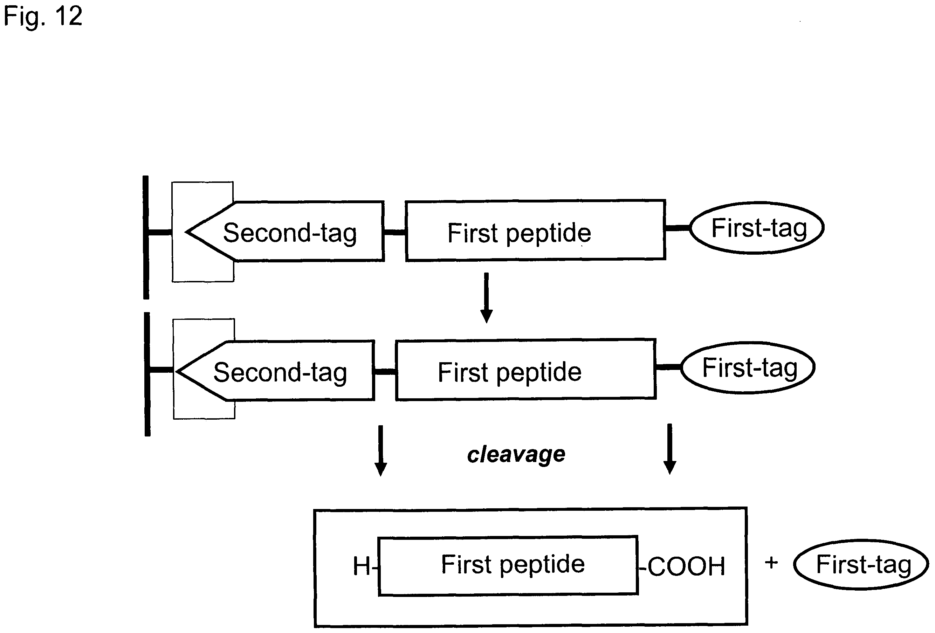

Fig. 12 is a schematic representation illustrating on-support cleavage of an internal peptide standard comprising both a first tag and a second tag. The internal peptide standard immobilized to a support via the second tag can be cleaved directly on the support subsequent to washing steps. These washing steps will remove side products of peptide synthesis which did not bind specifically or which did not form a covalent bond. The purified but still immobilized internal peptide standard can be cleaved on the support yielding the first peptide together with the first tag. The second tag is still bound to the support (not shown in the figure). The first tag could be used for quantification of the amount of the first peptide generated by the cleavage reaction.

Fig. 14 shows the composition of a second tag containing desthiobiotin. The composition of a second tag is shown using the three-letter-code for the naturally occurring amino acid residues. This illustrated second tag is an embodiment of a second tag optimized for cleavage using protease trypsin. Arginine residue is occupying the P1-position. The proline residue is known to be preferred by the protease trypsin in the P2-position. The alanine residue is well accepted in the P3-position. Desthiobiotin is used as a reactive moiety within the second tag enabling selective binding to supports modified with biotin/desthiobiotin binding proteins like avidin or streptavidin. To space apart the desthiobiotin residue from the amino acid residues and to allow effective interaction of the desthiobiotin with the binding pockets on the desthiobiotin binding support a linker molecule, Ttds, was introduced. The chemical structure of the linker is shown together with the chemical structure of desthiobiotin. The desthiobiotin is used instead of biotin because the affinity of desthiobiotin to the proteins streptavidin or avidin is much lower than the affinity of biotin. This enables elution of bound, desthiobiotin-containing internal peptide standards from the supports using biotin as a reagent effectively competing with the desthiobiotin-modified compounds.

Fig. 15 shows the composition of a first and second tag containing fluorescence label. The composition of a first tag and second tag is shown using the three-letter-code for the naturally occurring amino acid residues. The indicated first tag and second tag each represents a first and second tag optimized for cleavage using protease trypsin. In the first tag, serine and alanine residues are occupying the P1' and P2'-position, respectively. The fluorophore-containing amino acid residue Cal = β-7-methoxy-**C**oumaryl-**al**anine is optimized for the P3 '-position. In the P4'-position a glycine residue is used. In the second tag, an arginine residue is occupying the P1-position. The proline residue is known to be preferred by the protease trypsin in the P2-position. The Cal = β-7-methoxy-**C**oumaryl-**al**anine residue is well accepted in the P3-position in the case of trypsin as cleavage-mediating protease. The substituted coumaryl-residue in that alanine derivative yielded a highly fluorescent signal. Desthiobiotin is used as a reactive moiety within the second tag enabling selective binding to supports modified with biotin/desthiobiotin binding proteins like avidin or streptavidin. To space apart the desthiobiotin residue from the amino acid residues and to allow effective interaction of the desthiobiotin with the binding pockets on the desthiobiotin binding support a linker molecule, Ttds, was introduced. The chemical structure of the linker together with the chemical structure of desthiobiotin is shown in Fig. 14. Desthiobiotin is used instead of biotin because the affinity of desthiobiotin to the proteins streptavidin or avidin is much lower than the affinity of biotin. This enables elution of bound, desthiobiotin-containing internal peptide standards from the supports using biotin as a reagent effectively competing with the desthiobiotin-modified compounds.

Fig. 16 indicates various chemoselective chemical reactions which can be used for immobilization of peptide derivatives onto supports. Four different chemical reactions are shown which are suitable for chemoselective formation of a covalent bond between an internal peptide standard and an appropriately modified support. In reaction A), support bound aldehydes (R₁ = hydrogen) or ketones (R₁ is not hydrogen) and amino-oxy-moiety containing internal peptide standards react by formation of oxime-bonds. In reaction B), support bound mercaptans and maleinimide containing internal peptide standards react by formation of substituted succinimides. In reaction C), support bound hydrazides and aldehyde containing internal peptide standards react by formation of substituted hydrazones. In reaction D), support bound quinones and cyclopentadiene containing internal peptide standards react by formation of substituted Diels-Alder-Adducts.

Fig. 17 indicates further chemoselective chemical reactions which can be used for immobilization of peptide derivatives onto supports. Four different chemical reactions are shown which are suitable for chemoselective formation of a covalent bond between an internal peptide standard and an appropriately modified support. In reaction A), support bound aldehydes (R = hydrogen) or ketones (R is not hydrogen) and substituted hydroxyl-amines within the internal peptide standards react by formation of nitrones. In reaction B), support bound aldehydes (R= hydrogen) and aromatic amino carboxyl containing internal peptide standards react by formation of substituted 2,3,4a,8a-tetrahydro-1*H*-azoline-4-ones. In reaction C), support bound thioamides and alpha-bromo-ketone containing internal peptide standards react by formation of substituted thiazoles. In reaction D), support bound thioamides and substituted phenylene diamine containing internal peptide standards react by formation of substituted benzimidazoles.

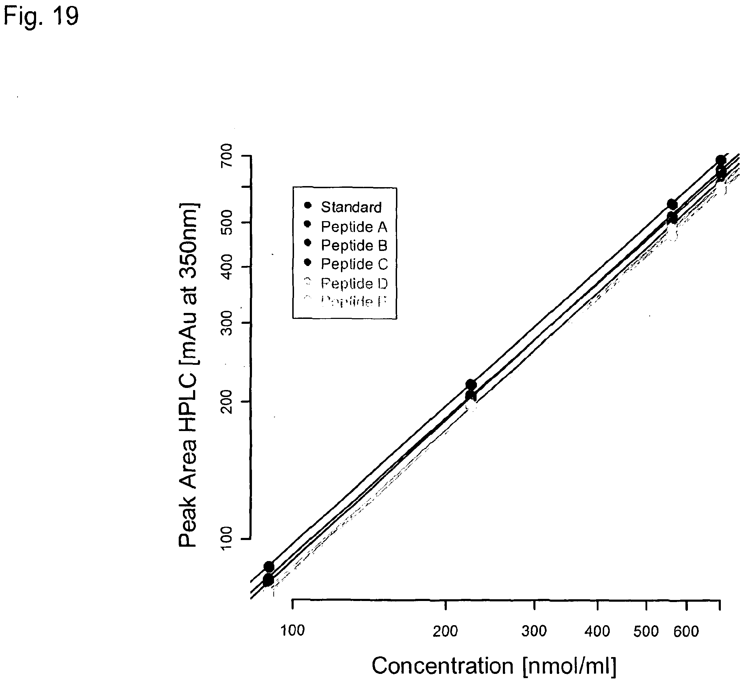

Fig. 19 shows straight calibration lines for the quantification of five different labeled peptides. All peptides carry the C-terminal quantification-tag -SA-nitroTyr-G-OH (Peptides A-E, Table 1) that allows quantification by UV measurement at 350 nm. Also the calibration line for the external standard Ac-SA-nitroTyr-R-OH is depicted.

### Example 1: Compatibility of chromophore incorporated into first tag with enzymatic cleavage of the internal peptide standard

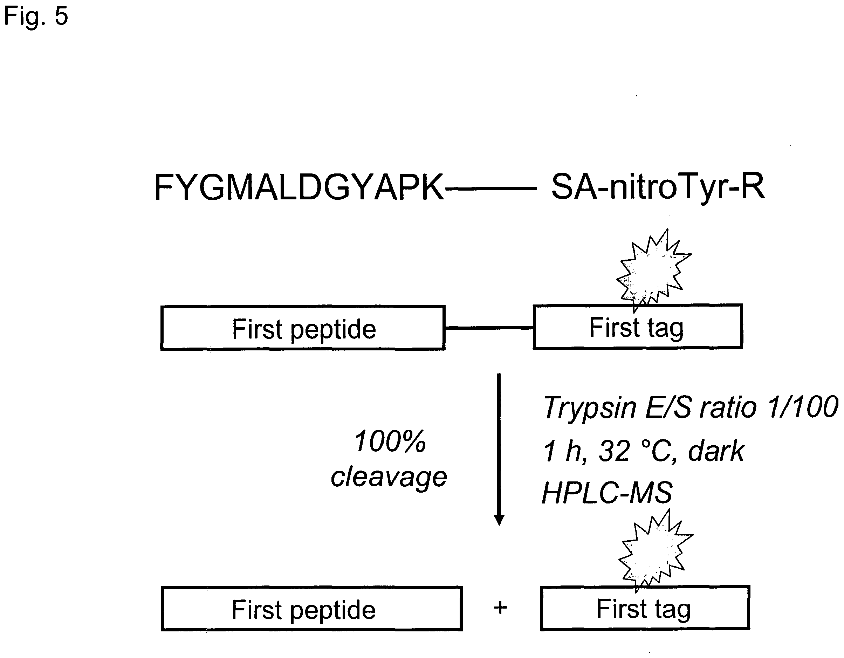

The basic experiment subject to this example is illustrated in Fig. 5.

The internal standard peptide of the given sequence indicated as the one letter code for the naturally occurring amino acid residues was synthesized in a stepwise manner on a solid support. The first tag contains a modified amino acid residue (nitroTyr = 3'-nitro-tyrosine) which yielded a specific UV-Vis absorption at wavelengths between 350-410 nm. The peptide was released from the solid support and treated with the protease trypsin for 1 hour at 32°C. Before and subsequent to the cleavage reaction a HPLC-MS analysis was performed to estimate the effectiveness of cleavage reaction. The basic experiment is illustrated in Fig. 5.

From this example it was concluded that the incorporation of a chromophore into a first tag is compatible with enzymatic cleavage of the internal peptide standard.

### Example 2: UV-Vis traces at 410 nm before and subsequent to cleavage of internal peptide standard

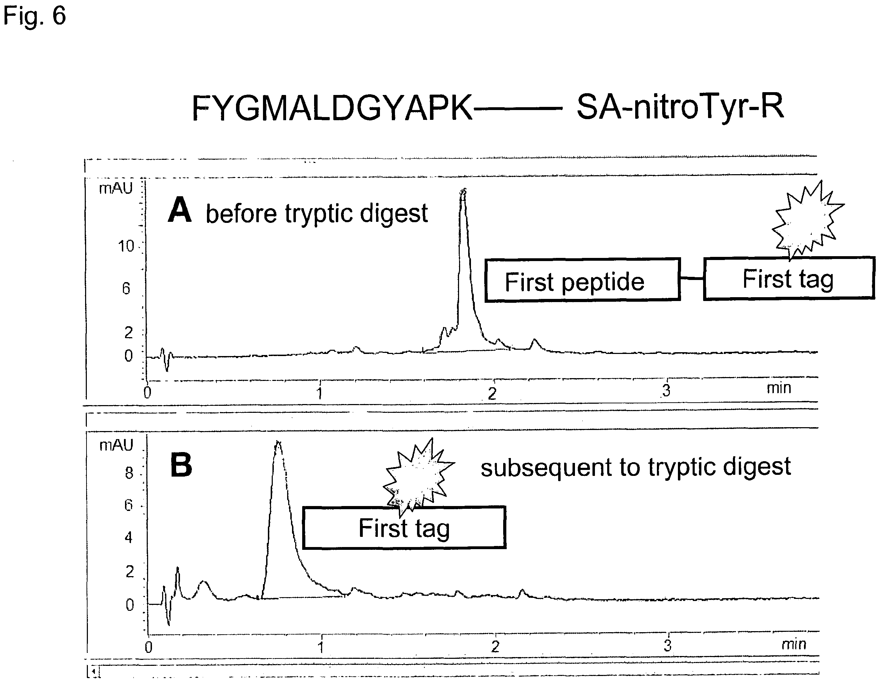

The internal peptide standard depicted in Fig. 7 was analysed using HPLC using the one letter code for the naturally occurring amino acid residues. The UV-Vis traces at 410 nm are shown. Fig. 6 A) indicates the UV-trace of the reaction solution before addition of the protease trypsin. The internal peptide standard containing the meta-nitro-tyrosine absorbing at 410 nm elutes from the PR-18 column with a retention time of about 1.85 minutes. Fig. 6 B) indicates the UV-trace of the reaction solution subsequent to addition of the protease trypsin and incubation of the reaction mixture at 32°C for one hour. The released first tag containing the meta-nitro-tyrosine absorbing at 410 nm elutes from the PR-18 column with a retention time of about 0.8 minutes. The released first peptide is not visible at that wavelength. Because there is nearly no remaining signal visible at retention time of about 1.85 minutes the cleavage reaction seems to be very effective with a cleavage rate close to 100%.

The result of the experiment underlying this example is illustrated in Fig. 6.

This result demonstrates that the meta-nitro-tyrosine residue in the first tag is compatible with cleavage reactions mediated by the protease trypsin.

### Example 3: UV-Vis spectra of acetylated first tag containing meta-nitro-tyrosine

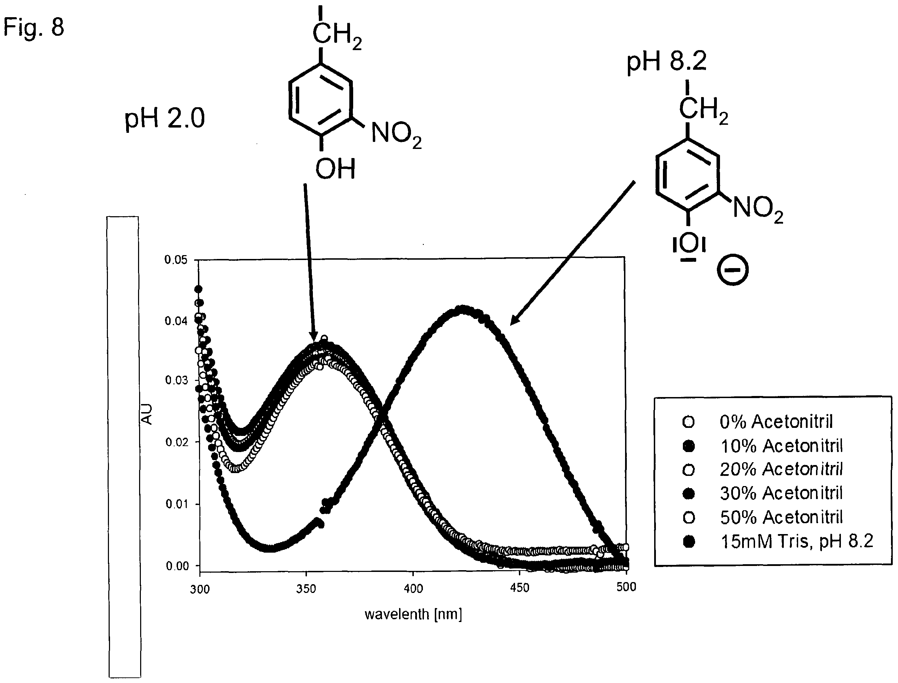

The UV-Vis spectra in the range of 300-500 nm are shown in Fig. 8 for acetylated first tag Ser-Ala-mNitroTyr-Arg. The spectra were recorded for solution of the tag in different acetonitrile/water mixtures containing trifluoroacetic acid. Because of the trifluoroacetic acid the pH value of these mixtures is in the range of pH 2. Additionally, a UV-Vis spectrum of the acetylated tag Ser-Ala-mNitroTyr-Arg dissolved in 15 mM TRIS buffer, pH 8.2, was recorded. The maximum of absorbance in the measured range is about 420 nm for pH 8.2 but about 355 nm for pH 2.0. The reason for that difference is the protonation state of the side chain of the meta-nitro-tyrosine residue. The different structures for the different protonation states are shown. On the left hand side the structure for the protonated meta-nitro-tyrosine residue is shown. This is the predominant moiety at pH 2.0. On the right hand side the structure for the dissociated meta-nitro-tyrosine residue is shown. This is the predominant moiety at pH 8.2 because of the increased acidic character of the phenolic group in proximity to the nitro function.

The very similar UV-Vis spectra at pH 2.0 for the different solvent mixtures enable quantification of first tags containing the meta-nitro-tyrosine using HPLC gradients. The total absorbance at 355 nm is nearly independent on the acetonitrile content of the solvent.

### Example 4: Quantification of non-purified internal peptide standard analysed by HPLC-MS

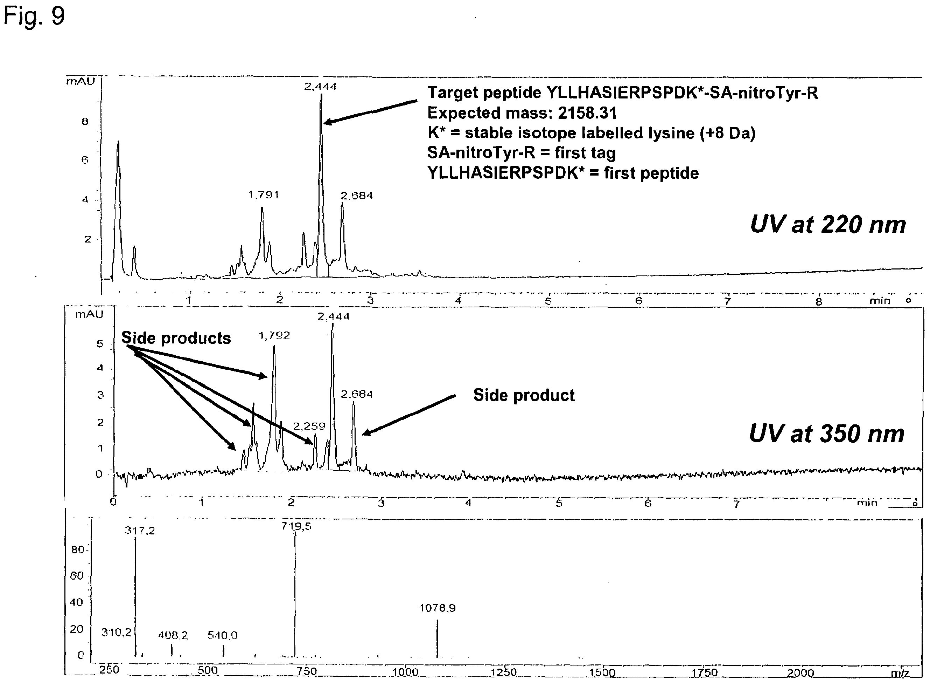

The result of this quantification is shown in Fig. 9.

The internal peptide standard is analysed by HPLC-ESI-MS equipped with a UV-Vis detector. On top of Fig. 9, the UV trace at 220 nm is shown. There are several signals representing different impurities besides the target internal peptide standard which could be identified in the ESI mass spectrum within the retention time region of 2.425-2.502 minutes. In the middle of Fig. 9, the UV-trace at 350 nm is shown which is indicative for the first tag which in this case is equipped with a chromophore (meta-nitro-tyrosine) absorbing specifically at 350 nm at pH value of 2. Comparing the signals in the UV-Vis trace at 220 nm with the signals in the UV-Vis trace at 350 nm it becomes clear that nearly all of the side products having the first tag. Nevertheless, the areas of the different peaks at 350 nm allow the exact quantification of each side product and of the target internal standard peptide if the HPLC-MS system was calibrated with a defined amount of meta-nitro-tyrosine-containing first tag.

### Example 5: Generation and use of an internal peptide standard comprising both a first and a second tag

More complex peptide standards can be produced using peptide synthesis as illustrated in Fig. 10. Subsequent to generation of the first tag on a solid support, either by stepwise synthesis or by attachment of a pre-synthesized tag directly to the solid support, a first peptide followed by a second tag are synthesized in a stepwise manner. Alternatively, the first peptide is attached to the first tag and then the second tag is attached to the bound first-peptide-first-tag structure. Additionally, combinations of stepwise synthesis and attachment of pre-synthesized structures are possible.

Upon release of the internal peptide standard by appropriate methods from the solid support, the internal peptide standard can be re-immobilized onto another support via moieties within the second tag. This immobilization reaction can be selected from a chemoselective formation of a chemical bond or from the specific binding of the second tag or part of the second tag to binding partners or interaction partners.

### Example 6: Methods for quantifying first peptides released by cleavage of purified internal peptide standards

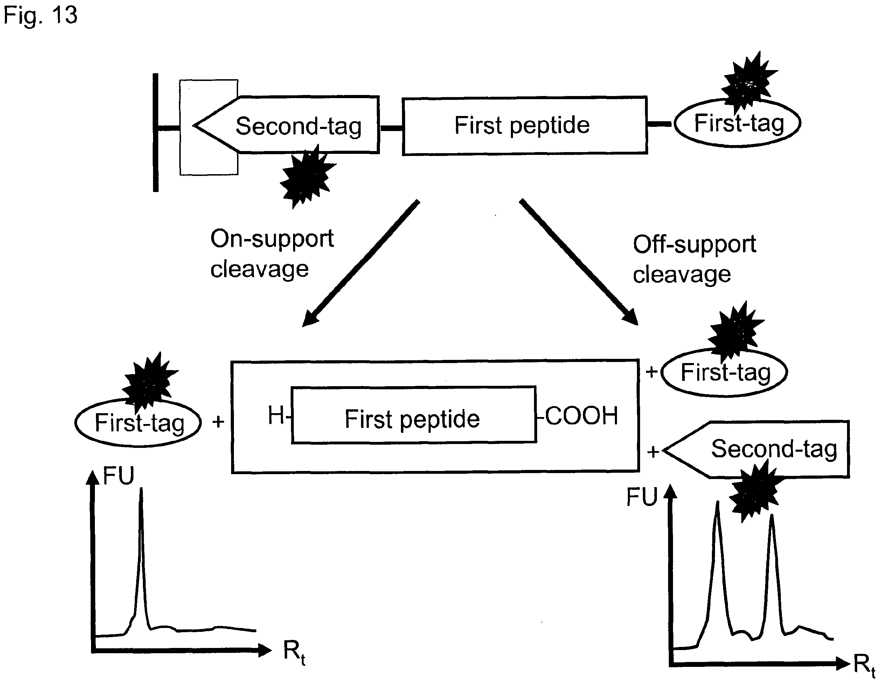

As illustrated in Fig. 13, an internal peptide standard comprising both a first tag and a second tag is immobilized to a support. Both the second tag and the first tag are labeled with a fluorescent amino acid residue. Subsequent to immobilization of the non-purified internal peptide standard, the support is washed to remove acetylated side products of peptide synthesis which did not bind specifically or which did not form a covalent bond. Subsequently, the purified internal peptide standard can be either cleaved directly on the support (left hand side of Fig. 13) or after release from the support (off-support cleavage, right hand side of Fig. 13). After on-support cleavage the second tag will remain on the support and the first peptide together with the first tag will be released from the support. The released mixture of first peptide and first tag can be analyzed by HPLC equipped with a fluorescence detector. If the system is calibrated with a fluorescent reference first tag, the fluorescence intensity (measured in fluorescence units, FU) of the released first tag can be used for quantification of the first tag and therefore for the quantification of the first peptide.

After off-support cleavage, the first peptide together with the first tag and the second tag will be released from the support. The released mixture of first peptide, first tag and second tag can be analyzed by HPLC equipped with a fluorescence detector. If the system was calibrated with a fluorescent reference first tag and or a fluorescent second tag, the fluorescence intensities, measured in fluorescence units, FU, of the released first tag and second tag could be used for quantification of the tags and therefore for the quantification of the first peptide.

### Example 7: Calibration of HPLC system using defined amounts of chromophore-containing first tag

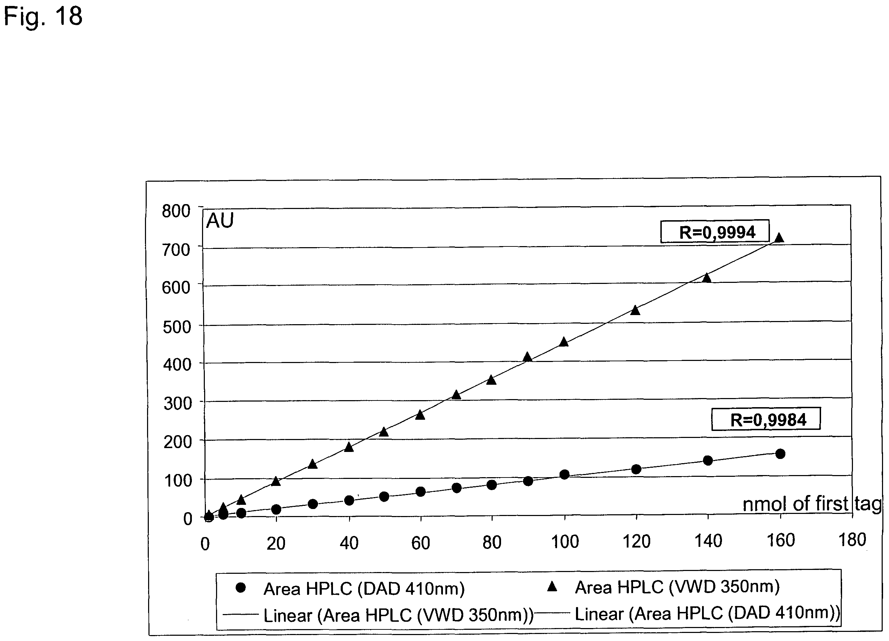

The acetylated form of a first tag (Ac-Ser-Ala-NitroTyr-Arg, see Fig. 7) was synthesized and absolutely quantified by amino acid analysis. Defined amounts of quantified, acetylated first tag were loaded onto the HPLC column and the resulting areas - absorption units, AU, of the peaks detected at either 350 nm or 410 nm were plotted against the total amount of the acetylated first tag. Linear regression analysis was performed for the two series of experiments yielding correlations of 0.9994 and 0.9986, respectively. The result is indicated in Fig. 18.

The linear correlation which is evident form Fig. 18 enables determination of amounts of meta-nitro-tyrosine-containing first tag released from appropriate internal peptide standards. As is also evident from Fig. 18, determining absorption at 350 nm is clearly advantageous compared to absorption at 410 nm in terms of the slope observed which allows a more accurate determination of the first tag.

### Example 8: Peptide quantifications with a nitro-Tyrosine-based tag: Linearity and independence from peptide sequence

Five different peptides carrying the C-terminal quantification-tag -SA-nitroTyr-G-OH (Peptides A-E, Table 1) were synthesized by Fmoc-based solid phase peptide synthesis and purified by HPLC. Figure 19 shows linear calibration curves for the quantification of the five peptides via HPLC in comparison to the external standard Ac-SA-nitroTyr-R-OH. Quantification was performed at 350 nm.

It turned out that the quantification was linear over the tested concentration range (100-700 nmol/ml) for all peptides and independently of the peptide sequence. The overall error (SD) for quantification was 5.4 %.

**Table 1: Peptides quantified by UV absorption of a nitro-Tyrosine-based quantification tag.**

| Name | Sequence |
|---|---|
| Standard | Ac-SA-nitroTyr-R-OH |
| Peptide A | H-LTGEVVALK-SA-nitroTyr-G-OH |
| Peptide B | H-LIDWGLAEFYHPAQEYNV-R-SA-nitroTyr-G-OH |
| Peptide C | H-EQSQPCADNAVLSSGLTAA-R-SA-nitroTyr-G-OH |
| Peptide D | H-ELIFQETA-R-SA-nitroTyr-G-OH |
| Peptide E | H-LAYTFEVQK-SA-nitroTyr-G-OH |

### Example 9: Peptide quantifications with a nitro-Tyrosine-based tag: Efficiency of tag cleavage from 23 different peptides

23 peptides carrying the C-terminal quantification tag -SA-nitroTyr-G-OH were synthesized by Fmoc-based solid phase peptide synthesis and purified by HPLC. Each peptide was subjected to the following trypsin cleavage protocol:
Trypsin Activation: Trypsin (20 µg, sequencing grade modified trypsin, Promega Corporation, Madison, WI, USA, catalogue no. V511, specific activity 16.965 u/mg) was dissolved in Trypsin resuspension buffer (200 µL, Promega, catalogue no. V542A, ingredient 50 mM acetic acid) and incubated for 15 min at 37 °C.
Reduction and alkylation: The peptide (approx. 0.05 mg) was dissolved in 1 M urea and 100 mM fresh ammonium hydrogen carbonate (300 µL). TCEP (tris(2-carboxyethyl) phosphine hydrochloride, 0.2 M, 7.5 µL, final concentration 5 mM) was added and the solution incubated at 37°C for 1 h. After addition of iodoacetamide (0.5 M, 6.0 µL, final concentration 10 mM) the solution was incubated at RT in the dark for another 30 min.
Digestion: The sample was digested with activated Trypsin solution (10 µL per well, 1.0 µg, resulting enzyme/substrate ratio 1:50) over night at RT. To stop the digestion, HCl (2 M, 8 µL, final concentration 50 mM) and TFA (3.2 µL, final concentration 1 %, pH<2) were added. Finally, the sample was analyzed by LC-MS at 350 nm.

Table 2 shows that the cleavage efficiency - as determined by LC-MS and UV detection at 350 nm - was >99 % in all cases, with the exception of peptide 20 (HLVSPEALDLLD-K-SA-nitroTyr-G) where it was only 75 %. This means that except for one peptide the cleavage of the quantification tag is basically quantitative.

Peptide 20 bears an aspartic acid attached to the C-terminal lysine. It is known that peptide bonds between Lys or Arg and the acidic amino acids Asp and Glu are often cleaved slowly by trypsin (Rehm, H.; Letzel, T. Der Experimentator Proteinbiochemie/Proteomics, 6th edition, 2010, Spektrum Akademischer Verlag, Heidelberg, p. 242). However, the incomplete cleavage of these bonds is not problematic for the application of tagged peptides in MS-based proteomics, since this information can be taken into account for the selection process of proteotypic peptides. Practically, this means that peptides with D-K- and E-K-bonds will simply not be chosen as proteotypic peptides for the detection and quantification of proteins.

The reduction and the alkylation process were applied to the test peptides, because it was aimed to subject the peptides to the same workflow as it is usually employed for the preparation of proteotypic peptides from proteins. This has the advantage that the tagged peptides can be spiked into biological samples prior to their cleavage by tryptic digest and thus are cleaved concomitantly with the protein. As all cleavage experiments led to the desired products, it can be concluded that the quantification tag did neither interfere with the reduction nor with the alkylation step.

**Table 2: Cleavage of the C-terminal quantification tag -SA-nitroTyr-G from 23 different peptides.**

| Pept # | Sequence | Percent Cleavage of Tag [by UV at 350nm] |
|---|---|---|
| 1 | IISIFSGTE-K-SA-nitroTyr-G | 100 |
| 2 | SVIDPVPAPVGDSHVDGAA-K-SA-nitroTyr-G | 100 |
| 3 | LGINLLGGPLGG-K-SA-nitroTyr-G | 100 |
| 4 | SPEVLLGSA-R-SA-nitroTyr-G | 100 |
| 5 | NLDENGLDLLS-K-SA-nitroTyr-G | 100 |
| 6 | LDTETEGVPSTAI-R-SA-nitroTyr-G | 100 |
| 7 | LTGEVVAL-K-SA-nitroTyr-G | 100 |
| 8 | TVGALQVLGTEAQSSLL-K-SA-nitroTyr-G | 100 |
| 9 | NLSSNEAISLEEI-R-SA-nitroTyr-G | 99.2 |
| 10 | HGDLPDIQI-K-SA-nitroTyr-G | 100 |
| 11 | AALSALESFL-K-SA-nitroTyr-G | 100 |
| 12 | SLGPPQGEEDSVP-R-SA-nitroTyr-G | 100 |
| 13 | GQVFDVGP-R-SA-nitroTyr-G | 99.4 |
| 14 | ELIFEETA-R-SA-nitroTyr-G | 99.6 |
| 15 | LDLDLTADSQPPVF-K-SA-nitroTyr-G | 100 |
| 16 | LSPPYSSPQEFAQDVG-R-SA-nitroTyr-G | 100 |
| 17 | DHQYQFLEDAV-R-SA-nitroTyr-G | 100 |
| | LPNGVFTPDFQEFVN-K-SA-nitroTyr-G | 95.4 |
| 18 | LGLEDFESL-K-SA-nitroTyr-G | 100 |
| 19 | LIDWGLAEFYHPAQEYNV-R-SA-nitroTyr-G | 100 |
| 20 | HLVSPEALDLLD-K-SA-nitroTyr-G | 74.7 |
| 21 | EQSQPC(Acm)ADNAVLSSGLTAA-R-SA-nitroTyr-G | 100 |
| 22 | ELIFQETA-R-SA-nitroTyr-G | 100 |
| 23 | LAYTFEVQ-K-SA-nitroTyr-G | 100 |

### Example 10: Peptide quantifications with a nitro-Tyrosine-based tag: Efficiency of tag cleavage from 38 different peptides

38 peptides carrying the N-terminal quantification tag Ac-nitroTyr-SGK- were synthesized by SPOT synthesis (Table 3). The peptide sequences are based on two different amino acid sequences, which in each case contain 19 different amino acids N-terminally attached to lysine (the 20 naturally occurring amino acids except cysteine).

All peptides were subjected to the same trypsin cleavage protocol as described in Example 9. The cleavage efficiency for the 38 peptides - as determined by LC-MS and UV detection at 350 nm - is shown in Table 3. It is apparent that the quantification tag is efficiently cleaved (>99 %) in nearly all cases.

Four peptides were not efficiently cleaved. These are the peptides containing a K-D- (peptides 3 and 23) and a K-P-bond (peptides 13 and 33), respectively. This is not surprising, as it is known that peptide bonds between Lys/Arg and Pro as well as the acidic amino acids Asp and Glu are often cleaved slowly by trypsin (Rehm, H.; Letzel, T. Der Experimentator Proteinbiochemie/Proteomics, 6th edition, 2010, Spektrum Akademischer Verlag, Heidelberg, p. 242). However, the incomplete cleavage of these bonds is not problematic for the application of tagged peptides in MS-based proteomics, since this information can be taken into account for the selection process of proteotypic peptides. Practically, this means that peptides with D-K-,E-K- and P-K-bonds will simply not be chosen as proteotypic peptides for the detection and quantification of proteins.

Peptides with a KK- or KR-motif were cleaved C-terminally to this group with good cleavage efficiencies (peptides 9, 15, 29, 35).

**Table 3: Cleavage of the N-terminal quantification tag Ac-nitroTyr-SGK- from 38 different peptides.**

| Peptide # | Sequence | Kusebauch 24h, RT: Percent Cleavage of Tag [by UV350 - Peptide-Tag vs. Tag] |
|---|---|---|
| 1 | Ac-nitroTyr-SGKATHYFIDLN-K-OH | 100.0 |
| 3 | Ac-nitroTyr-SGK**D**THYFIDLN-K-OH | **60.2** |
| 4 | Ac-nitroTyr-SGKETHYFIDLN-K-OH | 100.0 |
| 5 | Ac-nitroTyr-SGKFTHYFIDLN-K-OH | 99.0 |
| 6 | Ac-nitroTyr-SGKGTHYFIDLN-K-OH | 100.0 |
| 7 | Ac-nitroTyr-SGKHTHYFIDLN-K-OH | 100.0 |
| 8 | Ac-nitroTyr-SGKITHYFIDLN-K-OH | 100.0 |
| 9 | Ac-nitroTyr-SGKKTHYFIDLN-K-OH | 100.0 |
| 10 | Ac-nitroTyr-SGKLTHYFIDLN-K-OH | 100.0 |
| 11 | Ac-nitroTyr-SGKMTHYFIDLN-K-OH | 100.0 |
| 12 | Ac-nitroTyr-SGKNTHYFIDLN-K-OH | 100.0 |
| 13 | Ac-nitroTyr-SGK**P**THYFIDLN-K-OH | **37.0** |
| 14 | Ac-nitroTyr-SGKQTHYFIDLN-K-OH | 100.0 |
| 15 | Ac-nitroTyr-SGKRTHYFIDLN-K-OH | 100.0 |
| 16 | Ac-nitroTyr-SGKSTHYFIDLN-K-OH | 100.0 |
| 17 | Ac-nitroTyr-SGKTTHYFIDLN-K-OH | 100.0 |
| 18 | Ac-nitroTyr-SGKVTHYFIDLN-K-OH | 100.0 |
| 19 | Ac-nitroTyr-SGKWTHYFIDLN-K-OH | 100.0 |
| 20 | Ac-nitroTyr-SGKYTHYFIDLN-K-OH | 100.0 |
| 21 | Ac-nitroTyr-SGKAVALSLDDV-K-OH | 100.0 |
| 23 | Ac-nitroTyr-SGKDVALSLDDV-K-OH | **59.2** |
| 24 | Ac-nitroTyr-SGKEVALSLDDV-K-OH | 100.0 |
| 25 | Ac-nitroTyr-SGKFVALSLDDV-K-OH | 100.0 |
| 26 | Ac-nitroTyr-SGKGVALSLDDV-K-OH | 99.3 |
| 27 | Ac-nitroTyr-SGKHVALSLDDV-K-OH | 100.0 |
| 28 | Ac-nitroTyr-SGKIVALSLDDV-K-OH | 100.0 |
| 29 | Ac-nitroTyr-SGKKVALSLDDV-K-OH | 100.0 |
| 30 | Ac-nitroTyr-SGKLVALSLDDV-K-OH | 100.0 |
| 31 | Ac-nitroTyr-SGKMVALSLDDV-K-OH | 100.0 |
| 32 | Ac-nitroTyr-SGKNVALSLDDV-K-OH | 100.0 |
| 33 | Ac-nitroTyr-SGKPVALSLDDV-K-OH | **5.4** |
| 34 | Ac-nitroTyr-SGKQVALSLDDV-K-OH | 100.0 |
| 35 | Ac-nitroTyr-SGKRVALSLDDV-K-OH | 100.0 |
| 36 | Ac-nitroTyr-SGKSVALSLDDV-K-OH | 100.0 |
| 37 | Ac-nitroTyr-SGKTVALSLDDV-K-OH | 100.0 |
| 38 | Ac-nitroTyr-SGKVVALSLDDV-K-OH | 100.0 |
| 39 | Ac-nitroTyr-SGKWVALSLDDV-K-OH | 100.0 |
| 40 | Ac-nitroTyr-SGKYVALSLDDV-K-OH | 99.1 |

The features of the present invention disclosed in the specification, the claims, the sequence listing and/or the drawings may both separately and in any combination thereof be material for realizing the invention in various forms thereof.

## Claims

1. An internal peptide standard comprising a first tag to which is coupled a first peptide comprising a mass label and an amino acid sequence, wherein the C-terminal end of the amino acid sequence of the first peptide corresponds to the C-terminal end generated by a sequence-specific hydrolysis of an amide bond, ester bond or thioester bond of a peptide and wherein the first tag comprises a label and is removable by a means selected from the group comprising chemical cleavage, enzymatic cleavage and physical cleavage, the label comprising meta-nitro-tyrosine.

2. The internal peptide standard according to claim 1, wherein the removal of the first tag is sequence-specific.

3. The internal peptide standard according to claim 1 or 2, wherein the mass label is selected from the group comprising C isotopes and N isotopes.

4. The internal peptide standard according to any one of claims 1 to 3, which comprises a second tag.

5. The internal peptide standard according to claim 4, wherein the second tag comprises an anchor moiety.

6. The internal peptide standard according to claim 5, wherein the anchor moiety is selected from the group comprising biotin, desthiobiotin, avidin, streptavidin or is a chemical group allowing chemoselective reaction with the appropriate reactive function on the surface.

7. The internal peptide standard according to claim 5 or 6, wherein a linker connects the anchor moiety and the first peptide.

8. The internal peptide standard according to any one of claims 1 to 7, wherein the first tag is coupled to the C-terminal end of the first peptide.

9. The internal peptide standard according to any one of claims 1 to 8, wherein the second tag is coupled to the N-terminal end of the first peptide.

10. A composition comprising a plurality of different species of the internal peptide standard of any one of claims 1 to 9, wherein said different species of said internal peptide standard differ from each other in the first tag and/or in the amino acid sequence of the first peptide.

11. Use of the internal peptide standard according to any one of claims 1 to 9 in a method for determining the presence and/or quantity of a target polypeptide in at least one mixture of different polypeptides and/or quantity of a purified polypeptide.

12. A method for determining the presence and/or quantity of a target polypeptide of which a first peptide is a proteotypic peptide, in at least one mixture of different polypeptides, comprising:
a) providing a mixture of different polypeptides preferably containing the target polypeptide;
b) adding a quantity of (i) an internal peptide standard according to any one of claims 1 to 9 or (ii) a plurality of different species of said internal peptide standard, said different species of said internal peptide standard differing from each other in the first tag and/or in the amino acid sequence of the first peptide, thereby generating a spiked mixture;
c) treating the spiked mixture with a means for sequence-specific hydrolysis to generate a plurality of peptides from the mixture of different polypeptides and to cleave off the first tag from the internal peptide standard/standards thus releasing the first peptide, whereby the plurality of peptides comprises the proteotypic peptide derived from the different polypeptides and the first peptide released from the internal standard;
d) determining the amount of the first tag contained in the spiked mixture and therefrom the quantity of the internal standard/standards added in step b);
e) determining the ratio of the first peptide to the proteotypic peptide derived from the different polypeptides, each generated in step c); and
f) calculating from the ratio and the quantity of the internal standard/standards determined in step d) the quantity of the target polypeptide in the mixture of different polypeptides.

13. A method for determining the presence and/or quantity of a target polypeptide of which a first peptide is a proteotypic peptide, in at least one mixture of different polypeptides, comprising:
a) providing a mixture of different polypeptides preferably containing the target polypeptide;
b) providing (i) an internal peptide standard according to any one of claims 1 to 9 or (ii) a plurality of different species of said internal peptide standard, said different species of said internal peptide standard differing from each other in the first tag and/or in the amino acid sequence of the first peptide, treating the internal peptide standard or the plurality of different species of said internal peptide standard with a means for sequence-specific hydrolysis to cleave off the first tag from the internal peptide standard or the plurality of different species of said internal peptide standard thus releasing the first peptide;
either
ca1) adding the reaction mixture obtained in step b) to the mixture of different polypeptides of step a) and
ca2) treating the mixture obtained in step ca1) with a means for sequence-specific hydrolysis to generate a plurality of peptides from the mixture of different polypeptides, whereby the plurality of peptides comprises the proteotypic peptide;
or
cb1) treating the mixture of different polypeptides of step a) with a means for sequence-specific hydrolysis to generate a plurality of peptides from the mixture of different polypeptides, whereby the plurality of peptides comprises the proteotypic peptide; and
cb2) adding the reaction mixture obtained in step b) to the mixture obtained in step cb1)
whereby after step ca2) and cb2), respectively, a spiked mixture is obtained;
d) determining the amount of the first tag contained in the spiked mixture and therefrom the quantity of the internal standard/standards added in step ca1) or cb2);
e) determining in the spiked mixture the ratio of the first peptide to the proteotypic peptide derived from the different polypeptides; and
f) calculating from the ratio and the quantity of the internal standard/standards determined in step d) the quantity of the target polypeptide in the mixture of different polypeptides.

14. A method for determining the presence and/or quantity of a target polypeptide of which a first peptide is a proteotypic peptide, in at least one mixture of different polypeptides, comprising:
a) providing (i) an internal peptide standard according to any one of claims 1 to 9 or (ii) a plurality of different species of said internal peptide standard, said different species of said internal peptide standard differing from each other in the first tag and/or in the amino acid sequence of the first peptide, treating the internal peptide standard or the plurality of different species of said internal peptide standard with a means for sequence-specific hydrolysis to cleave off the first tag from the internal peptide standard or the plurality of different species of said internal peptide standard thus releasing the first peptide;
b) optionally removing the means for sequence-specific hydrolysis;
c) determining the amount of the first tag contained in the mixture obtained from step a) or b) and therefrom the amount of the internal standard contained in said mixture;
d) providing a mixture of different polypeptides preferably containing the target polypeptide;
either
ea1) adding a part of the reaction mixture obtained in step a) or b) to the mixture of different polypeptides of step d) and
ea2) treating the mixture obtained in step ea1) with a means for sequence-specific hydrolysis to generate a plurality of peptides from the mixture of different polypeptides, whereby the plurality of peptides comprises the proteotypic peptide;
or
eb1) treating the mixture of different polypeptides of step d) with a means for sequence-specific hydrolysis to generate a plurality of peptides from the mixture of different polypeptides, whereby the plurality of peptides comprises the proteotypic peptide; and
eb2) adding a part of the mixture obtained in step a) or b) to the mixture obtained in step eb1);
whereby after step ea2) and eb2), respectively, a spiked mixture is obtained;
f) determining in the spiked mixture the ratio of the first peptide to the proteotypic peptide derived from the different polypeptides; and
g) calculating from the ratio and the quantity of the internal standard/standards the quantity of the target polypeptide in the mixture of different polypeptides.

15. The method according to any one of claims 12 to 14, wherein the ratio of the first peptide released from the internal peptide standard to the proteotypic peptide derived from the different polypeptides is determined by mass spectrometry, preferably multistage mass spectrometry.

16. The method according to any one of claims 12 to 15, wherein the means for sequence-specific hydrolysis is selected from a proteolytic enzyme, a sequence-specific chemical reaction or a sequence-specific physical treatment.

## Patentansprüche

1. Interner Peptidstandard, umfassend ein erstes Tag, an das ein erstes Peptid gekoppelt ist, das eine Massenmarkierung und eine Aminosäuresequenz umfasst, wobei das C-terminale Ende der Aminosäuresequenz des ersten Peptids dem C-terminalen Ende entspricht, das durch eine sequenzspezifische Hydrolyse einer Amidbindung, Esterbindung oder Thioesterbindung eines Peptids erzeugt wird, und wobei das erste Tag eine Markierung umfasst und mit einem Mittel entfernbar ist, das aus der Gruppe ausgewählt ist, die eine chemische Spaltung, eine enzymatische Spaltung und eine physikalische Spaltung umfasst, wobei die Markierung meta-Nitrotyrosin umfasst.

2. Interner Peptidstandard gemäß Anspruch 1, wobei die Entfernung des ersten Tags sequenzspezifisch ist.

3. Interner Peptidstandard gemäß Anspruch 1 oder 2, wobei die Massenmarkierung aus der Gruppe ausgewählt ist, die C-Isotope und N-Isotope umfasst.

4. Interner Peptidstandard gemäß einem jeglichen der Ansprüche 1 bis 3, der einen zweiten Tag umfasst.

5. Interner Peptidstandard gemäß Anspruch 4, wobei das zweite Tag einen Ankerrest umfasst.

6. Interner Peptidstandard gemäß Anspruch 5, wobei der Ankerrest aus der Gruppe, die Biotin, Desthiobiotin, Avidin, Streptavidin umfasst, ausgewählt ist oder eine chemische Gruppe ist, die eine chemoselektive Reaktion mit der geeigneten reaktiven Funktion auf der Oberfläche ermöglicht.

7. Interner Peptidstandard gemäß Anspruch 5 oder 6, wobei ein Linker den Ankerrest und das erste Peptid verbindet.

8. Interner Peptidstandard gemäß einem jeglichen der Ansprüche 1 bis 7, wobei das erste Tag an das C-terminale Ende des ersten Peptids gekoppelt ist.

9. Interner Peptidstandard gemäß einem jeglichen der Ansprüche 1 bis 8, wobei das zweite Tag an das N-terminale Ende des ersten Peptids gekoppelt ist.

10. Zusammensetzung, umfassend eine Vielzahl verschiedener Spezies des internen Peptidstandards gemäß einem jeglichen der Ansprüche 1 bis 9, wobei sich die verschiedenen Spezies des internen Peptidstandards voneinander in dem ersten Tag und/oder in der Aminosäuresequenz des ersten Peptids unterscheiden.

11. Verwendung des internen Peptidstandards gemäß einem jeglichen der Ansprüche 1 bis 9 in einem Verfahren zum Bestimmen des Vorhandenseins und/oder der Menge eines Zielpolypeptids in mindestens einem Gemisch verschiedener Polypeptide und/oder Menge eines aufgereinigten Polypeptids.

12. Verfahren zum Bestimmen des Vorhandenseins und/oder der Menge eines Zielpolypeptids, von dem ein erstes Peptid ein proteotypisches Peptid ist, in mindestens einem Gemisch verschiedener Polypeptide, umfassend:
a) Bereitstellen eines Gemisches verschiedener Polypeptide, das vorzugsweise das Zielpolypeptid enthält;
b) Zugeben einer Menge (i) eines internen Peptidstandards gemäß einem jeglichen der Ansprüche 1 bis 9 oder (ii) einer Vielzahl verschiedener Spezies des internen Peptidstandards, wobei sich die verschiedenen Spezies des internen Peptidstandards voneinander in dem ersten Tag und/oder in der Aminosäuresequenz des ersten Peptids unterscheiden, wodurch ein angereichertes Gemisch erzeugt wird;
c) Behandeln des angereicherten Gemisches mit einem Mittel für eine sequenzspezifische Hydrolyse zur Erzeugung einer Vielzahl von Peptiden aus dem Gemisch verschiedener Polypeptide und zum Abspalten des ersten Tags von dem/den internen Peptidstandard/Peptidstandards, wodurch das erste Peptid freigesetzt wird und die Vielzahl von Peptiden das proteotypische Peptid, das von den verschiedenen Polypeptiden abstammt, und das erste Peptid, das von dem internen Standard freigesetzt wurde, umfasst;
d) Bestimmen der Menge des in dem angereicherten Gemisch enthaltenen ersten Tags und daraus der Menge des/der internen Standards, der/die in Schritt b) zugesetzt wurde/wurden;
e) Bestimmen des Verhältnisses des ersten Peptids zu dem proteotypischen Peptid, das von den verschiedenen Polypeptiden abstammt, das jeweils in Schritt c) erzeugt wurde; und
f) Berechnen aus dem Verhältnis und der Menge des/der internen Standards, die in Schritt d) bestimmt wurde, der Menge des Zielpolypeptids in dem Gemisch verschiedener Polypeptide.

13. Verfahren zum Bestimmen des Vorhandenseins und/oder der Menge eines Zielpolypeptids, von dem ein erstes Peptid ein proteotypisches Peptid ist, in mindestens einem Gemisch verschiedener Polypeptide, umfassend:
a) Bereitstellen eines Gemisches verschiedener Polypeptide, das vorzugsweise das Zielpolypeptid enthält;
b) Bereitstellen (i) eines internen Peptidstandards gemäß einem jeglichen der Ansprüche 1 bis 9 oder (ii) einer Vielzahl verschiedener Spezies des internen Peptidstandards, wobei sich die verschiedenen Spezies des internen Peptidstandards voneinander in dem ersten Tag und/oder in der Aminosäuresequenz des ersten Peptids unterscheiden, Behandeln des internen Peptidstandards oder der Vielzahl verschiedener Spezies des internen Peptidstandards mit einem Mittel für eine sequenzspezifische Hydrolyse zum Abspalten des ersten Tags von dem internen Peptidstandard oder der Vielzahl verschiedener Spezies des internen Peptidstandards, wodurch das erste Peptid freigesetzt wird;
entweder
ca1) Zugeben des in Schritt b) erhaltenen Reaktionsgemisches zu dem Gemisch verschiedener Polypeptide von Schritt a) und
ca2) Behandeln des in Schritt ca1) erhaltenen Gemisches mit einem Mittel für eine sequenzspezifische Hydrolyse zur Erzeugung einer Vielzahl von Peptiden aus dem Gemisch verschiedener Polypeptide, wodurch die Vielzahl von Peptiden das proteotypische Peptid umfasst;
oder
cb1) Behandeln des Gemisches verschiedener Polypeptide von Schritt a) mit einem Mittel für eine sequenzspezifische Hydrolyse zur Erzeugung einer Vielzahl von Peptiden aus dem Gemisch verschiedener Polypeptide, wodurch die Vielzahl von Peptiden das proteotypische Peptid umfasst; und
cb2) Zugeben des in Schritt b) erhaltenen Reaktionsgemisches zu dem in Schritt cb1) erhaltenen Gemisch;
wodurch nach Schritt ca2) bzw. cb2) ein angereichertes Gemisch erhalten wird;
d) Bestimmen der Menge des in dem angereicherten Gemisch enthaltenen ersten Tags und daraus der Menge des/der internen Standards, der/die in Schritt ca1) oder cb2) zugesetzt wurde/wurden;
e) Bestimmen in dem angereicherten Gemisch des Verhältnisses des ersten Peptids zu dem proteotypischen Peptid, das von den verschiedenen Polypeptiden abstammt; und
f) Berechnen aus dem Verhältnis und der Menge des/der internen Standards, die in Schritt d) bestimmt wurde, der Menge des Zielpolypeptids in dem Gemisch verschiedener Polypeptide.

14. Verfahren zum Bestimmen des Vorhandenseins und/oder der Menge eines Zielpolypeptids, von dem ein erstes Peptid ein proteotypisches Peptid ist, in mindestens einem Gemisch verschiedener Polypeptide, umfassend:
a) Bereitstellen (i) eines internen Peptidstandards gemäß einem jeglichen der Ansprüche 1 bis 9 oder (ii) einer Vielzahl verschiedener Spezies des internen Peptidstandards, wobei sich die verschiedenen Spezies des internen Peptidstandards voneinander in dem ersten Tag und/oder in der Aminosäuresequenz des ersten Peptids unterscheiden, Behandeln des internen Peptidstandards oder der Vielzahl verschiedener Spezies des internen Peptidstandards mit einem Mittel für eine sequenzspezifische Hydrolyse zum Abspalten des ersten Tags von dem internen Peptidstandard oder der Vielzahl verschiedener Spezies des internen Peptidstandards, wodurch das erste Peptid freigesetzt wird;
b) gegebenenfalls Entfernen des Mittels für eine sequenzspezifische Hydrolyse;
c) Bestimmen der Menge des ersten Tags, das in dem aus Schritt a) oder b) erhaltenen Gemisch enthalten ist, und daraus der Menge des in dem Gemisch enthaltenen internen Standards;
d) Bereitstellen eines Gemisches verschiedener Polypeptide, das vorzugsweise das Zielpolypeptid enthält;
entweder
ea1) Zugeben eines Teils des in Schritt a) oder b) erhaltenen Reaktionsgemisches zu dem Gemisch verschiedener Polypeptide von Schritt d) und
ea2) Behandeln des in Schritt ea1) erhaltenen Gemisches mit einem Mittel für eine sequenzspezifische Hydrolyse zur Erzeugung einer Vielzahl von Peptiden aus dem Gemisch verschiedener Polypeptide, wodurch die Vielzahl von Peptiden das proteotypische Peptid umfasst;
oder
eb1) Behandeln des Gemisches verschiedener Polypeptide von Schritt d) mit einem Mittel für eine sequenzspezifische Hydrolyse zur Erzeugung einer Vielzahl von Peptiden aus dem Gemisch verschiedener Polypeptide, wodurch die Vielzahl von Peptiden das proteotypische Peptid umfasst; und
eb2) Zugeben eines Teils des in Schritt a) oder b) erhaltenen Gemisches zu dem in Schritt eb1) erhaltenen Gemisches;
wodurch nach Schritt ea2) bzw. eb2) ein angereichertes Gemisch erhalten wird;
f) Bestimmen in dem angereicherten Gemisch des Verhältnisses des ersten Peptids zu dem proteotypischen Peptid, das von den verschiedenen Polypeptiden abstammt; und
g) Berechnen aus dem Verhältnis und der Menge des/der internen Standards der Menge des Zielpolypeptids in dem Gemisch verschiedener Polypeptide.

15. Verfahren gemäß einem jeglichen der Ansprüche 12 bis 14, wobei das Verhältnis des von dem internen Peptidstandard freigesetzen ersten Peptids zu dem aus den verschiedenen Polypeptiden abstammenden proteotypischen Peptid durch Massenspektrometrie, vorzugsweise mehrstufige Massenspektrometrie bestimmt wird.

16. Verfahren gemäß einem jeglichen der Ansprüche 12 bis 15, wobei das Mittel für eine sequenzspezifische Hydrolyse aus einem proteolytischen Enzym, einer sequenzspezifischen chemischen Reaktion oder einer sequenzspezifischen physikalischen Behandlung ausgewählt wird.

## Revendications

1. Etalon peptidique interne comprenant un premier marquage auquel est couplé un premier peptide comprenant un marqueur de masse et une séquence d'acides aminés, où l'extrémité C-terminale de la séquence d'acides aminés du premier peptide correspond à l'extrémité C-terminale générée par une hydrolyse, spécifique pour une séquence, d'une liaison amide, d'une liaison ester ou d'une liaison thioester d'un peptide et où le premier marquage comprend un marqueur et est éliminable par un moyen choisi dans le groupe comprenant clivage chimique, clivage enzymatique et clivage physique, le marqueur comprenant de la méta-nitro-tyrosine.

2. Etalon peptidique interne selon la revendication 1, dans lequel l'élimination du premier marquage est spécifique pour une séquence.

3. Etalon peptidique interne selon la revendication 1 ou 2, dans lequel le marqueur de masse est choisi dans le groupe comprenant des isotopes de C et des isotopes de N.

4. Etalon peptidique interne selon l'une quelconque des revendications 1 à 3, qui comprend un deuxième marquage.

5. Etalon peptidique interne selon la revendication 4, dans lequel le deuxième marquage comprend une portion d'ancrage.

6. Etalon peptidique interne selon la revendication 5, dans lequel la portion d'ancrage est choisie dans le groupe comprenant biotine, desthiobiotine, avidine, streptavidine ou est un groupe chimique premettant une réaction chimiosélective avec la fonction réactive appropriée sur la surface.

7. Etalon peptidique interne selon la revendication 5 ou 6, dans lequel un lieur relie la portion d'ancrage et le premier peptide.

8. Etalon peptidique interne selon l'une quelconque des revendications 1 à 7, dans lequel le premier marquage est couplé à l'extrémité C-terminale du premier peptide.

9. Etalon peptidique interne selon l'une quelconque des revendications 1 à 8, dans lequel le deuxième marquage est couplé à l'extrémité N-terminale du premier peptide.

10. Composition comprenant une pluralité de différentes espèces de l'étalon peptidique interne selon l'une quelconque des revendications 1 à 9, où lesdites espèces différentes dudit étalon peptidique interne diffèrent l'une de l'autre dans le premier marquage et/ou dans la séquence d'acides aminés du premier peptide.

11. Utilisation de l'étalon peptidique interne selon l'une quelconque des revendications 1 à 9 dans un procédé pour la détermination de la présence et/ou la quantité d'un polypeptide cible dans au moins un mélange de différents polypeptides et/ou la quantité d'un polypeptide purifié.

12. Procédé pour la détermination de la présence et/ou la quantité d'un polypeptide cible dont un premier peptide est un peptide protéotypique, dans au moins un mélange de différents polypeptides, comprenant:
a) la fourniture d'un mélange de différents polypeptides contenant de préférence le polypeptide cible;
b) l'addition d'une quantité de (i) un étalon peptidique interne selon l'une quelconque des revendications 1 à 9 ou (ii) une pluralité d'espèces différentes dudit étalon peptidique interne, lesdites espèces différentes dudit étalon peptidique interne différant l'une de l'autre dans le premier marquage et/ou dans la séquence d'acides aminés du premier peptide, générant ainsi un mélange surchargé;
c) le traitement du mélange surchargé avec un moyen pour l'hydrolyse spécifique de la séquence pour générer une pluralité de peptides à partir du mélange de différents polypeptides et pour séparer par clivage le premier marquage du/des étalon(s) peptidique(s) interne(s) libérant ainsi le premier peptide, tandis que la pluralité de peptides comprend ainsi le peptide protéotypique dérivé des différents polypeptides et le premier peptide libéré de l'étalon interne;
d) la détermination de la quantité du premier marquage contenue dans le mélange surchargé et à partir de là la quantité de l'étalon/des étalons interne(s) ajoutée dans l'étape b);
e) la détermination du rapport du premier peptide au peptide protéolytique dérivé des différents polypeptides, chacun généré dans l'étape c); et
f) le calcul à partir du rapport et de la quantité de l'étalon/des étalons interne(s) déterminé(s) dans l'étape d) de la quantité du polypeptide cible dans le mélange de différents polypeptides.

13. Procédé pour la détermination de la présence et/ou de la quantité d'un polypeptide cible dont un premier peptide est un peptide protéotypique, dans au moins un mélange de différents polypeptides, comprenant:
a) la fourniture d'un mélange de différents polypeptides contenant de préférence le polypeptide cible;
b) la fourniture (i) d'un étalon peptidique interne selon l'une quelconque des revendications 1 à 9 ou (ii) une pluralité d'espèces différentes dudit étalon peptidique interne, lesdites espèces différentes dudit étalon peptidique interne différant l'une de l'autre dans le premier marquage et/ou dans la séquence d'acides aminés du premier peptide, le traitement de l'étalon peptidique interne ou de la pluralité d'espèces différentes dudit étalon peptidique interne avec un moyen pour l'hydrolyse spécifique de la séquence pour séparer par clivage le premier marquage de l'étalon peptidique interne ou la pluralité d'espèces différentes dudit étalon peptidique interne libérant ainsi le premier peptide;
soit
ca1) l'addition du mélange réactionnel obtenu dans l'étape b) au mélange de différents polypeptides de l'étape a) et
ca2) le traitement du mélange obtenu dans l'étape ca1) avec un moyen pour l'hydrolyse spécifique de la séquence pour générer une pluralité de peptides à partir du mélange de différents polypeptides, tandis que la pluralité de peptides comprend ainsi le peptide protéotypique;
soit
cb1) le traitement du mélange de différents polypeptides de l'étape a) avec un moyen pour l'hydrolyse spécifique de la séquence pour générer une pluralité de peptides à partir du mélange de différents polypeptides, tandis que la pluralité de peptides comprend ainsi le peptide protéotypique; et
cb2) l'addition du mélange réactionnel obtenu dans l'étape b) au mélange obtenu dans l'étape cb1);
tandis qu'on obtient après l'étape ca2) et cb2), respectivement, un mélange surchargé;
d) la détermination de la quantité du premier marquage contenue dans le mélange surchargé et à partir de là la quantité du(des) étalon(s) interne(s) ajoutée dans l'étape ca1) ou cb2);
e) la détermination dans le mélange surchargé du rapport du premier peptide au peptide protéotypique dérivé des différents polypeptides; et
f) le calcul à partir du rapport et de la quantité du(des) étalon(s) interne(s) dans l'étape d) de la quantité du polypeptide cible dans le mélange de différents polypeptides.

14. Procédé pour la détermination de la présence et/ou de la quantité d'un poylpeptide cible dont un premier peptide est un peptide protéotypique, dans au moins un mélange de différents polypeptides, comprenant:
a) la fourniture (i) d'un étalon peptidique interne selon l'une quelconque des revendications 1 à 9 ou (ii) une pluralité d'espèces différentes dudit étalon peptidique interne, lesdites espèces différentes dudit étalon peptidique interne différant l'une de l'autre dans le premier marquage et/ou dans la séquence d'acides aminés du premier peptide, le traitement de l'étalon peptidique interne ou de la pluralité d'espèces différentes dudit étalon peptidique interne avec un moyen pour l'hydrolyse spécifique de la séquence pour séparer par clivage le premier marquage de l'étalon peptidique interne ou de la pluralité d'espèces différentes dudit étalon peptidique interne libérant ainsi le premier peptide;
b) en option, la suppression du moyen pour l'hydrolyse spécifique de la séquence;
c) la détermination de la quantité du premier marquage contenue dans le mélange obtenu à partir de l'étape a) ou b) et à partir de là la quantité de l'étalon interne contenue dans ledit mélange;
d) la fourniture d'un mélange de différents polypeptides contenant de préférence le polypeptide cible;
soit
ea1) l'addition d'une partie du mélange de réaction obtenu dans l'étape a) ou b) au mélange de différents polypeptides de l'étape d) et
ea2) le traitement du mélange obtenu dans l'étape ea1) avec un moyen pour l'hydrolyse spécifique de la séquence afin de générer une pluralité de peptides à partir du mélange de différents polypeptides, tandis que la pluralité de peptides comprend le peptide protéotypique;
soit
eb1) le traitement du mélange de polypeptides différents de l'étape d) avec un moyen pour l'hydrolyse spécifique de la séquence pour générer une pluralité de peptides à partir du mélange de différents polypeptides, la pluralité de peptides comprenant ainsi le peptide protéotypique; et
eb2) l'addition d'une partie du mélange obtenu dans l'étape a) ou b) au mélange obtenu dans l'étape eb1);
tandis que, après l'étape ea2) et eb2), respectivement, on obtient un mélange surchargé;
f) la détermination dans le mélange surchargé du rapport du premier peptide au peptide protéotypique dérivé des différents polypeptides; et
g) le calcul à partir du rapport et de la quantité du ou des étalon(s) interne(s) la quantité du polypeptide cible dans le mélange de différents polypeptides.

15. Procédé selon l'une quelconque des revendications 12 à 14, dans lequel le rapport du premier peptide libéré à partir de l'étalon peptidique interne sur le peptide protéotypique dérivé des différents polypeptides est déterminé par spectrométrie de masse, de préférence spectrométrie de masse multi-stades.

16. Procédé selon l'une quelconque des revendications 12 à 15, dans lequel le moyen pour l'hydrolyse spécifique de la séquence est choisi parmi une enzyme protéolytique, une réaction chimique spécifique de la séquence ou un traitement physique spécifique de la séquence.
